# EUROPEAN PATENT SPECIFICATION

(11) **EP 1 216 306 B1**
(45) Date of publication and mention of the grant of the patent: **09.07.2008**
(21) Application number: 00954228.3
(22) Date of filing: 23.08.2000
(51) Int. Cl.: C12N 15/82, C12N 15/12, C12N 5/10, C12P 21/02, A01H 5/00

(54) **COMMERCIAL PRODUCTION OF CHYMOSIN IN PLANTS**
KOMMERZIELLE HERSTELLUNG VON CHYMOSIN IN PFLANZEN
PRODUCTION AU NIVEAU INDUSTRIEL DU TAUX DE CHYMOSINE DANS DES PLANTES

(30) Priority: 23.08.1999 US 378696
(43) Date of publication of application: 26.06.2002
(73) Proprietor: SemBioSys Genetics Inc., Calgary, Alberta T1Y 7L3 (CA)
(72) Inventor: VAN ROOIJEN, Gijs, Calgary, Alberta T2L 1T1 (CA); KEON, Richard, Glenn, Calgary, Alberta T2E 1R1 (CA); BOOTHE, Joseph, Calgary, Alberta T2M 0T7 (CA); SHEN, Yin, Calgary, Alberta T2L 1J4 (CA)
(74) Representative: Williams, Aylsa
(86) International application number: PCT/CA2000/000975
(87) International publication number: WO 2001/014571

(56) References cited:
- WO-A-92/01042
- WO-A-92/18634
- WO-A-98/49326
- DD-A- 265 164
- US-A- 5 714 474

### SEQUENCE LISTING

<110> van Rooijen, Gijs
   Keon, Richard Glenn
   Boothe, Joseph
   Shen, Yin
   SemBioSys Genetics Inc.
<120> Commercial Production of Chymosin in Plants
<130> 9369-152
<140>
   <141>
<160> 4
<170> PatentIn Ver. 2.0
<210> 1
   <211> 1173
   <212> DNA
   <213> Bovine
<220>
   <221> CDS
   <222> (1)..(1173)
<400> 1
   atg aac ttc ctt aag tct ttc cct ttc tac gct ttc ctt tgt ttc ggt    48
   Met Asn Phe Leu Lys Ser Phe Pro Phe Tyr Ala Phe Leu Cys Phe Gly
     1               5                  10                  15
   caa tac ttc gtt gct gtt act cac gct gct gag atc acc cgc att cct    96
   Gln Tyr Phe Val Ala Val Thr His Ala Ala Glu Ile Thr Arg Ile Pro
                20                  25                  30
   ctc tac aaa ggt aag tct ctc cgt aag gcg ctg aag gaa cat gga ctt   144
   Leu Tyr Lys Gly Lys Ser Leu Arg Lys Ala Leu Lys Glu His Gly Leu
            35                  40                  45
   cta gaa gac ttc ttg cag aaa caa cag tat ggc atc agc agc aag tac   192
   Leu Glu Asp Phe Leu Gln Lys Gln Gln Tyr Gly Ile Ser Ser Lys Tyr
        50                  55                  60
   tcc ggc ttc ggt gaa gtt gct agc gtg cca ctt acc aac tac ctt gat   240
   Ser Gly Phe Gly Glu Val Ala Ser Val Pro Leu Thr Asn Tyr Leu Asp
    65                  70                  75                  80
   agt caa tac ttt ggg aag atc tac ctc gga acc ccg cct caa gag ttc   288
   Ser Gln Tyr Phe Gly Lys Ile Tyr Leu Gly Thr Pro Pro Gln Glu Phe
                    85                  90                  95
   acc gtt ctc ttt gat act ggt tcc tct gac ttc tgg gtt ccc tct atc   336
   Thr Val Leu Phe Asp Thr Gly Ser Ser Asp Phe Trp Val Pro Ser Ile
               100                 105                 110
   tac tgc aag agc aat gcc tgc aag aac cac caa aga ttc gat ccg aga   384
   Tyr Cys Lys Ser Asn Ala Cys Lys Asn His Gln Arg Phe Asp Pro Arg
           115                 120                 125
   aag tcg tcc acc ttc cag aac tta ggc aaa ccc ttg tct ata cac tac   432
   Lys Ser Ser Thr Phe Gln Asn Leu Gly Lys Pro Leu Ser Ile His Tyr
       130                 135                 140
   ggt aca ggt agc atg caa gga atc tta ggc tat gat acc gtc act gtc   480
   Gly Thr Gly Ser Met Gln Gly Ile Leu Gly Tyr Asp Thr Val Thr Val
   145                 150                 155                 160
   tcc aac att gtg gac att caa cag aca gta gga ctt agc acc caa gaa   528
   Ser Asn Ile Val Asp Ile Gln Gln Thr Val Gly Leu Ser Thr Gln Glu
                   165                 170                 175
   cca ggt gat gtc ttc acc tat gca gaa ttc gat ggc atc ctt ggt atg   576
   Pro Gly Asp Val Phe Thr Tyr Ala Glu Phe Asp Gly Ile Leu Gly Met
               180                 185                 190
   gca tac cca tcg ctc gcg tca gag tac tcg ata cct gtg ttt gac aac   624
   Ala Tyr Pro Ser Leu Ala Ser Glu Tyr Ser Ile Pro Val Phe Asp Asn
           195                 200                 205
   atg atg aac cga cac cta gta gct caa gac ttg ttc tcg gtt tac atg   672
   Met Met Asn Arg His Leu Val Ala Gln Asp Leu Phe Ser Val Tyr Met
       210                 215                 220
   gac agg aat ggc cag gag agc atg ctc acg ctt gga gct att gat cca   720
   Asp Arg Asn Gly Gln Glu Ser Met Leu Thr Leu Gly Ala Ile Asp Pro
   225                 230                 235                 240
   tcc tac tac aca gga tct ctt cac tgg gtt cca gtc act gtg cag cag   768
   Ser Tyr Tyr Thr Gly Ser Leu His Trp Val Pro Val Thr Val Gln Gln
                   245                 250                 255
   tac tgg caa ttc act gtg gac agt gtc acc atc agc ggt gtg gtt gtt   816
   Tyr Trp Gln Phe Thr Val Asp Ser Val Thr Ile Ser Gly Val Val Val
               260                 265                 270
   gca tgt gaa ggt gga tgt caa gct atc ttg gat acc ggt acg tcc aag   864
   Ala Cys Glu Gly Gly Cys Gln Ala Ile Leu Asp Thr Gly Thr Ser Lys
           275                 280                 285
   ctg gtc gga cct agc agc gac att ctc aac att cag caa gct att gga   912
   Leu Val Gly Pro Ser Ser Asp Ile Leu Asn Ile Gln Gln Ala Ile Gly
       290                 295                 300
   gcc aca cag aac cag tac ggt gag ttt gac ata gat tgc gac aac ctt   960
   Ala Thr Gln Asn Gln Tyr Gly Glu Phe Asp Ile Asp Cys Asp Asn Leu
   305                 310                 315                 320
   agc tac atg cct aca gtt gtc ttt gag atc aac ggc aag atg tac cca   1008
   Ser Tyr Met Pro Thr Val Val Phe Glu Ile Asn Gly Lys Met Tyr Pro
                   325                 330                 335
   ctg acc ccc tcc gcc tat acc agc cag gat caa ggg ttc tgc acc agt   1056
   Leu Thr Pro Ser Ala Tyr Thr Ser Gln Asp Gln Gly Phe Cys Thr Ser
               340                 345                 350
   gga ttc cag agt gag aac cat tcc cag aaa tgg atc ttg gga gat gtg   1104
   Gly Phe Gln Ser Glu Asn His Ser Gln Lys Trp Ile Leu Gly Asp Val
           355                 360                 365
   ttc att cgt gag tac tac agc gtc ttt gac agg gcc aac aac ctc gtt   1152
   Phe Ile Arg Glu Tyr Tyr Ser Val Phe Asp Arg Ala Asn Asn Leu Val
       370                 375                 380
   ggg cta gct aaa gca atc tga                                       1173
   Gly Leu Ala Lys Ala Ile
   385                 390
<210> 2
   <211> 390
   <212> PRT
   <213> Bovine
<400> 2
   Met Asn Phe Leu Lys Ser Phe Pro Phe Tyr Ala Phe Leu Cys Phe Gly
   1               5                   10                  15
   Gln Tyr Phe Val Ala Val Thr His Ala Ala Glu Ile Thr Arg Ile Pro
               20                  25                  30
   Leu Tyr Lys Gly Lys Ser Leu Arg Lys Ala Leu Lys Glu His Gly Leu
           35                  40                  45
   Leu Glu Asp Phe Leu Gln Lys Gln Gln Tyr Gly Ile Ser Ser Lys Tyr
       50                  55                  60
   Ser Gly Phe Gly Glu Val Ala Ser Val Pro Leu Thr Asn Tyr Leu Asp
   65                  70                  75                  80
   Ser Gln Tyr Phe Gly Lys Ile Tyr Leu Gly Thr Pro Pro Gln Glu Phe
                   85                  90                  95
   Thr Val Leu Phe Asp Thr Gly Ser Ser Asp Phe Trp Val Pro Ser Ile
               100                 105                 110
   Tyr Cys Lys Ser Asn Ala Cys Lys Asn His Gln Arg Phe Asp Pro Arg
           115                 120                 125
   Lys Ser Ser Thr Phe Gln Asn Leu Gly Lys Pro Leu Ser Ile His Tyr
       130                 135                 140
   Gly Thr Gly Ser Met Gln Gly Ile Leu Gly Tyr Asp Thr Val Thr Val
   145                 150                 155                 160
   Ser Asn Ile Val Asp Ile Gln Gln Thr Val Gly Leu Ser Thr Gln Glu
                   165                 170                 175
   Pro Gly Asp Val Phe Thr Tyr Ala Glu Phe Asp Gly Ile Leu Gly Met
               180                 185                 190
   Ala Tyr Pro Ser Leu Ala Ser Glu Tyr Ser Ile Pro Val Phe Asp Asn
           195                 200                 205
   Met Met Asn Arg His Leu Val Ala Gln Asp Leu Phe Ser Val Tyr Met
       210                 215                 220
   Asp Arg Asn Gly Gln Glu Ser Met Leu Thr Leu Gly Ala Ile Asp Pro
   225                 230                 235                 240
   Ser Tyr Tyr Thr Gly Ser Leu His Trp Val Pro Val Thr Val Gln Gln
                   245                 250                 255
   Tyr Trp Gln Phe Thr Val Asp Ser Val Thr Ile Ser Gly Val Val Val
               260                 265                 270
   Ala Cys Glu Gly Gly Cys Gln Ala Ile Leu Asp Thr Gly Thr Ser Lys
           275                 280                 285
   Leu Val Gly Pro Ser Ser Asp Ile Leu Asn Ile Gln Gln Ala Ile Gly
       290                 295                 300
   Ala Thr Gln Asn Gln Tyr Gly Glu Phe Asp Ile Asp Cys Asp Asn Leu
   305                 310                 315                 320
   Ser Tyr Met Pro Thr Val Val Phe Glu Ile Asn Gly Lys Met Tyr Pro
                   325                 330                 335
   Leu Thr Pro Ser Ala Tyr Thr Ser Gln Asp Gln Gly Phe Cys Thr Ser
               340                 345                 350
   Gly Phe Gln Ser Glu Asn His Ser Gln Lys Trp Ile Leu Gly Asp Val
           355                 360                 365
   Phe Ile Arg Glu Tyr Tyr Ser Val Phe Asp Arg Ala Asn Asn Leu Val
       370                 375                 380
   Gly Leu Ala Lys Ala Ile
   385                 390
<210> 3
   <211> 3957
   <212> DNA
   <213> Artificial Sequence
<220>
   <221> CDS
   <222> (1554)..(2726)
<220>
   <223> Description of Artificial Sequence: Figure 2
<400> 3
   ctgcaggaat tcattgtact cccagtatca ttatagtgaa agttttggct ctctcgccgg 60
   tggtttttta cctctattta aaggggtttt ccacctaaaa attctggtat cattctcact 120
   ttacttgtta ctttaatttc tcataatctt tggttgaaat tatcacgctt ccgcacacga 180
   tatccctaca aatttattat ttgttaaaca ttttcaaacc gcataaaatt ttatgaagtc 240
   ccgtctatct ttaatgtagt ctaacatttt catattgaaa tatataattt acttaatttt 300
   agcgttggta gaaagcataa agatttattc ttattcttct tcatataaat gtttaatata 360
   caatataaac aaattcttta ccttaagaag gatttcccat tttatatttt aaaaatatat 420
   ttatcaaata tttttcaacc acgtaaatct cataataata agttgtttca aaagtaataa 480
   aatttaactc cataattttt ttattcgact gatcttaaag caacacccag tgacacaact 540
   agccattttt ttctttgaat aaaaaaatcc aattatcatt gtattttttt tatacaatga 600
   aaatttcacc aaacaatcat ttgtggtatt tctgaagcaa gtcatgttat gcaaaattct 660
   ataattccca tttgacacta cggaagtaac tgaagatctg cttttacatg cgagacacat 720
   cttctaaagt aattttaata atagttacta tattcaagat ttcatatatc aaatactcaa 780
   tattacttct aaaaaattaa ttagatataa ttaaaatatt acttttttaa ttttaagttt 840
   aattgttgaa tttgtgacta ttgatttatt attctactat gtttaaattg ttttatagat 900
   agtttaaagt aaatataagt aatgtagtag agtgttagag tgttacccta aaccataaac 960
   tataacattt atggtggact aattttcata tatttcttat tgcttttacc ttttcttggt 1020
   atgtaagtcc gtaactagaa ttacagtggg ttgccatggc actctgtggt cttttggttc 1080
   atgcatgggt cttgcgcaag aaaaagacaa agaacaaaga aaaaagacaa aacagagaga 1140
   caaaacgcaa tcacacaacc aactcaaatt agtcactggc tgatcaagat cgccgcgtcc 1200
   atgtatgtct aaatgccatg caaagcaaca cgtgcttaac atgcacttta aatggctcac 1260
   ccatctcaac ccacacacaa acacattgcc tttttcttca tcatcaccac aaccacctgt 1320
   atatattcat tctcttccgc cacctcaatt tcttcacttc aacacacgtc aacctgcata 1380
   tgcgtgtcat cccatgccca aatctccatg catgttccaa ccaccttctc tcttatataa 1440
   tacctataaa tacctctaat atcactcact tctttcatca tccatccatc cagagtacta 1500
   ctactctact actataatac cccaacccaa ctcatattca atactactct act atg    1556
                                                                 Met
                                                                  1
   aac ttc ctt aag tct ttc cct ttc tac gct ttc ctt tgt ttc ggt caa    1604
   Asn Phe Leu Lys Ser Phe Pro Phe Tyr Ala Phe Leu Cys Phe Gly Gln
               5                  10                  15
   tac ttc gtt gct gtt act cac gct gct gag atc acc cgc att cct ctc    1652
   Tyr Phe Val Ala Val Thr His Ala Ala Glu Ile Thr Arg Ile Pro Leu
           20                  25                  30
   tac aaa ggt aag tct ctc cgt aag gcg ctg aag gaa cat gga ctt cta    1700
   Tyr Lys Gly Lys Ser Leu Arg Lys Ala Leu Lys Glu His Gly Leu Leu
       35                  40                  45
   gaa gac ttc ttg cag aaa caa cag tat ggc atc agc agc aag tac tcc    1748
   Glu Asp Phe Leu Gln Lys Gln Gln Tyr Gly Ile Ser Ser Lys Tyr Ser
    50                  55                  60                  65
   ggc ttc ggt gaa gtt gct agc gtg cca ctt acc aac tac ctt gat agt    1796
   Gly Phe Gly Glu Val Ala Ser Val Pro Leu Thr Asn Tyr Leu Asp Ser
                   70                  75                  80
   caa tac ttt ggg aag atc tac ctc gga acc ccg cct caa gag ttc acc    1844
   Gln Tyr Phe Gly Lys Ile Tyr Leu Gly Thr Pro Pro Gln Glu Phe Thr
               85                  90                  95
   gtt ctc ttt gat act ggt tcc tct gac ttc tgg gtt ccc tct atc tac    1892
   Val Leu Phe Asp Thr Gly Ser Ser Asp Phe Trp Val Pro Ser Ile Tyr
           100                 105                 110
   tgc aag agc aat gcc tgc aag aac cac caa aga ttc gat ccg aga aag    1940
   Cys Lys Ser Asn Ala Cys Lys Asn His Gln Arg Phe Asp Pro Arg Lys
       115                 120                 125
   tcg tcc acc ttc cag aac tta ggc aaa ccc ttg tct ata cac tac ggt    1988
   Ser Ser Thr Phe Gln Asn Leu Gly Lys Pro Leu Ser Ile His Tyr Gly
   130                 135                 140                 145
   aca ggt agc atg caa gga atc tta ggc tat gat acc gtc act gtc tcc    2036
   Thr Gly Ser Met Gln Gly Ile Leu Gly Tyr Asp Thr Val Thr Val Ser
                   150                 155                 160
   aac att gtg gac att caa cag aca gta gga ctt agc acc caa gaa cca    2084
   Asn Ile Val Asp Ile Gln Gln Thr Val Gly Leu Ser Thr Gln Glu Pro
               165                 170                 175
   ggt gat gtc ttc acc tat gca gaa ttc gat ggc atc ctt ggt atg gca    2132
   Gly Asp Val Phe Thr Tyr Ala Glu Phe Asp Gly Ile Leu Gly Met Ala
           180                 185                 190
   tac cca tcg ctc gcg tca gag tac tcg ata cct gtg ttt gac aac atg   2180
   Tyr Pro Ser Leu Ala Ser Glu Tyr Ser Ile Pro Val Phe Asp Asn Met
       195                 200                 205
   atg aac cga cac cta gta gct caa gac ttg ttc tcg gtt tac atg gac   2228
   Met Asn Arg His Leu Val Ala Gln Asp Leu Phe Ser Val Tyr Met Asp
   210                 215                 220                 225
   agg aat ggc cag gag agc atg ctc acg ctt gga gct att gat cca tcc   2276
   Arg Asn Gly Gln Glu Ser Met Leu Thr Leu Gly Ala Ile Asp Pro Ser
                   230                 235                 240
   tac tac aca gga tct ctt cac tgg gtt cca gtc act gtg cag cag tac   2324
   Tyr Tyr Thr Gly Ser Leu His Trp Val Pro Val Thr Val Gln Gln Tyr
               245                 250                 255
   tgg caa ttc act gtg gac agt gtc acc atc agc ggt gtg gtt gtt gca   2372
   Trp Gln Phe Thr Val Asp Ser Val Thr Ile Ser Gly Val Val Val Ala
           260                 265                 270
   tgt gaa ggt gga tgt caa gct atc ttg gat acc ggt acg tcc aag ctg   2420
   Cys Glu Gly Gly Cys Gln Ala Ile Leu Asp Thr Gly Thr Ser Lys Leu
       275                 280                 285
   gtc gga cct agc agc gac att ctc aac att cag caa gct att gga gcc   2468
   Val Gly Pro Ser Ser Asp Ile Leu Asn Ile Gln Gln Ala Ile Gly Ala
   290                 295                 300                 305
   aca cag aac cag tac ggt gag ttt gac ata gat tgc gac aac ctt agc   2516
   Thr Gln Asn Gln Tyr Gly Glu Phe Asp Ile Asp Cys Asp Asn Leu Ser
                   310                 315                 320
   tac atg cct aca gtt gtc ttt gag atc aac ggc aag atg tac cca ctg   2564
   Tyr Met Pro Thr Val Val Phe Glu Ile Asn Gly Lys Met Tyr Pro Leu
               325                 330                 335
   acc ccc tcc gcc tat acc agc cag gat caa ggg ttc tgc acc agt gga   2612
   Thr Pro Ser Ala Tyr Thr Ser Gln Asp Gln Gly Phe Cys Thr Ser Gly
           340                 345                 350
   ttc cag agt gag aac cat tcc cag aaa tgg atc ttg gga gat gtg ttc   2660
   Phe Gln Ser Glu Asn His Ser Gln Lys Trp Ile Leu Gly Asp Val Phe
       355                 360                 365
   att cgt gag tac tac agc gtc ttt gac agg gcc aac aac ctc gtt ggg   2708
   Ile Arg Glu Tyr Tyr Ser Val Phe Asp Arg Ala Asn Asn Leu Val Gly
   370                 375                 380                 385
   cta gct aaa gca atc tga agcttaataa gtatgaacta aaatgcatgt          2756
   Leu Ala Lys Ala Ile
                   390
   aggtgtaaga gctcatggag agcatggaat attgtatccg accatgtaac agtataataa 2816
   ctgagctcca tctcacttct tctatgaata aacaaaggat gttatgatat attaacactc 2876
   tatctatgca ccttattgtt ctatgataaa tttcctctta ttattataaa tcatctgaat 2936
   cgtgacggct tatggaatgc ttcaaatagt acaaaaacaa atgtgtacta taagactttc 2996
   taaacaattc taactttagc attgtgaacg agacataagt gttaagaaga cataacaatt 3056
   ataatggaag aagtttgtct ccatttatat attatatatt acccacttat gtattatatt 3116
   aggatgttaa ggagacataa caattataaa gagagaagtt tgtatccatt tatatattat 3176
   atactaccca tttatatatt atacttatcc acttatttaa tgtctttata aggtttgatc 3236
   catgatattt ctaatatttt agttgatatg tatatgaaag ggtactattt gaactctctt 3296
   actctgtata aaggttggat catccttaaa gtgggtctat ttaattttat tgcttcttac 3356
   agataaaaaa aaaattatga gttggtttga taaaatattg aaggatttaa aataataata 3416
   aataataaat aacatataat atatgtatat aaatttatta taatataaca tttatctata 3476
   aaaaagtaaa tattgtcata aatctataca atcgtttagc cttgctggac gactctcaat 3536
   tatttaaacg agagtaaaca tatttgactt tttggttatt taacaaatta ttatttaaca 3596
   ctatatgaaa tttttttttt ttatcggcaa ggaaataaaa ttaaattagg agggacaatg 3656
   gtgtgtccca atccttatac aaccaacttc cacaggaagg tcaggtcggg gacaacaaaa 3716
   aaacaggcaa gggaaatttt ttaatttggg ttgtcttgtt tgctgcataa tttatgcagt 3776
   aaaacactac acataaccct tttagcagta gagcaatggt tgaccgtgtg cttagcttct 3836
   tttattttat ttttttatca gcaaagaata aataaaataa aatgagacac ttcagggatg 3896
   tttcaaccct tatacaaaac cccaaaaaca agtttcctag caccctacca actaaggtac 3956
   c                                                                 3957
<210> 4
   <211> 390
   <212> PRT
   <213> Artificial Sequence
<400> 4
   Met Asn Phe Leu Lys Ser Phe Pro Phe Tyr Ala Phe Leu Cys Phe Gly
    1               5                  10                  15
   Gln Tyr Phe Val Ala Val Thr His Ala Ala Glu Ile Thr Arg Ile Pro
               20                  25                  30
   Leu Tyr Lys Gly Lys Ser Leu Arg Lys Ala Leu Lys Glu His Gly Leu
           35                  40                  45
   Leu Glu Asp Phe Leu Gln Lys Gln Gln Tyr Gly Ile Ser Ser Lys Tyr
       50                  55                  60
   Ser Gly Phe Gly Glu Val Ala Ser Val Pro Leu Thr Asn Tyr Leu Asp
   65                  70                  75                  80
   Ser Gln Tyr Phe Gly Lys Ile Tyr Leu Gly Thr Pro Pro Gln Glu Phe
                   85                  90                  95
   Thr Val Leu Phe Asp Thr Gly Ser Ser Asp Phe Trp Val Pro Ser Ile
               100                 105                 110
   Tyr Cys Lys Ser Asn Ala Cys Lys Asn His Gln Arg Phe Asp Pro Arg
           115                 120                 125
   Lys Ser Ser Thr Phe Gln Asn Leu Gly Lys Pro Leu Ser Ile His Tyr
       130                 135                 140
   Gly Thr Gly Ser Met Gln Gly Ile Leu Gly Tyr Asp Thr Val Thr Val
   145                 150                 155                 160
   Ser Asn Ile Val Asp Ile Gln Gln Thr Val Gly Leu Ser Thr Gln Glu
                   165                 170                 175
   Pro Gly Asp Val Phe Thr Tyr Ala Glu Phe Asp Gly Ile Leu Gly Met
               180                 185                 190
   Ala Tyr Pro Ser Leu Ala Ser Glu Tyr Ser Ile Pro Val Phe Asp Asn
           195                 200                 205
   Met Met Asn Arg His Leu Val Ala Gln Asp Leu Phe Ser Val Tyr Met
       210                 215                 220
   Asp Arg Asn Gly Gln Glu Ser Met Leu Thr Leu Gly Ala Ile Asp Pro
   225                 230                 235                 240
   Ser Tyr Tyr Thr Gly Ser Leu His Trp Val Pro Val Thr Val Gln Gln
                   245                 250                 255
   Tyr Trp Gln Phe Thr Val Asp Ser Val Thr Ile Ser Gly Val Val Val
               260                 265                 270
   Ala Cys Glu Gly Gly Cys Gln Ala Ile Leu Asp Thr Gly Thr Ser Lys
           275                 280                 285
   Leu Val Gly Pro Ser Ser Asp Ile Leu Asn Ile Gln Gln Ala Ile Gly
       290                 295                 300
   Ala Thr Gln Asn Gln Tyr Gly Glu Phe Asp Ile Asp Cys Asp Asn Leu
   305                 310                 315                 320
   Ser Tyr Met Pro Thr Val Val Phe Glu Ile Asn Gly Lys Met Tyr Pro
                   325                 330                 335
   Leu Thr Pro Ser Ala Tyr Thr Ser Gln Asp Gln Gly Phe Cys Thr Ser
               340                 345                 350
   Gly Phe Gln Ser Glu Asn His Ser Gln Lys Trp Ile Leu Gly Asp Val
           355                 360                 365
   Phe Ile Arg Glu Tyr Tyr Ser Val Phe Asp Arg Ala Asn Asn Leu Val
       370                 375                 380
   Gly Leu Ala Lys Ala Ile
   385                 390

## Description

### FIELD OF THE INVENTION

The present invention relates to improved methods for the recombinant production and isolation of chymosin from plants.

### BACKGROUND OF THE INVENTION

Chymosin, also known as rennin, is a commercially important enzymatic protein, commonly used in the cheese manufacturing industry to coagulate milk. Traditionally chymosin has been prepared from its natural source, the fourth stomach of unweaned calves, although recovery from the stomachs of other mammals, such as lamb, goats etc. heretofore was known. More recently, primarily as a result of a decrease in calf production, recombinant DNA techniques have been employed to produce chymosin by fermentation in genetically engineered microorganisms. Thus a variety of bacterial and fungal hosts have been genetically modified to produce chymosin by fermentation, including for example, the bacterial hosts *Escherichia coli,* (European Patent 0 134 662 A1; Nishimori et al. (1982) J. Biochem 91: 1085-1088.), *Bacillus subtilis* (US patent 5,624,819; 5,716,807 and Parente et al. (1991) FEMS 77: 243-250) and the fungal hosts *Aspergillus* sp. (European Patent 0 575 462 B1; US patents 5,364,770 and 5,863,759; Cullen et al. (1987) Bio/Technology 5: 369-375., Dunn-Coleman et al. (1991) Bio/Technology 9: 976-981., and Tsuchiua et al. (1993) Appl. Microbial Biotech. 40: 327-332), *Kluyveromyces lactis* (van der Berg et al. (1990) Bio/Technology 8: 135-139 and *Trichoderma ressei* (Jarkki et al.. (1989) Bio/Technology 7: 596-603; Pitts et al. (1991) Biochemical Society Transactions 19: 663-665). As well, more general expression in fungi, yeast and bacteria (US Patents 4,666,847) and in filamentous fungi (US patent 5,578,463).

The active enzyme chymosin (E.C. 3.4.23.4) is comprised of a polypeptide chain of a molecular mass of 35.6 kDa. However crude extracts of calf stomach mucosa in addition to active chymosin, contain two inactive precursor polypeptides known as pre-pro-chymosin and pro-chymosin. Pre-pro-chymosin contains an extra 58 amino acids at the N-terminus, whereas pro-chymosin contains an extra 42 amino acids. Conversion of the inactive precursor protein into enzymatically active chymosin requires the step-wise removal of the chymosin pre-peptide and pro-peptide. *In vivo* these activation steps take place in the calf stomach. The chymosin pre-peptide directs secretion of the polypeptide by the stomach cells and is removed upon secretion of the polypeptide by the stomach cells. The chymosin pro-peptide is subsequently removed in the gastric lumen, thereby activating the enzyme. The activation reaction can also be performed *in vitro* at pH values below 5. With regards to the enzyme chymosin, it should further be noted that chymosin purified from calf stomach is a mixture of two different polypeptides known as chymosin A and chymosin B. Both of these polypeptides are active and differ only with respect to one amino acid. The amino acid residue at position #290 is an aspartate residue in chymosin A and a glycine residue in chymosin B (Foltman et al., (1977) Proc. Natl. Acad. Sci. USA 74: 2331-2324; Foltman et al., (1979) J. Biol. Chem. 254: 8447-8456).

There are several disadvantages associated with the recombinant production of chymosin in fermentation systems. In general, fermentation systems require the use of large fermentation vessels that have both large space and energy requirements and consequently are costly. As well, the growth media require large volumes of water and may require special chemicals. Both of these may present environmental issues in the disposal of the large amounts of potentially harmful waste. Further, storage and shipment of raw material containing chymosin is problematic. The bacterial or fungal fermentation broth need to be processed immediately or refrigerated in large volumes since the enzyme is not stable for long periods in the broth.

The use of plants as bioreactors for the commercial production of recombinant proteins is well known. For example, avidin, β-glucuronidase and aprotinin (see patents US Patents 5,767,379, 5,804,694 and 5,824,870) have been recombinantly expressed in corn. Further, US Patents 5,543,576 and 5,714,474 are broadly directed to the recombinant production of enzymes in seeds and to the use of seeds or milled seeds comprising enzymes as a raw material in the preparation of food and feed products. Although US Patents 5,543,576 and 5,714,474 suggest chymosin as one potential enzyme that may be produced in seeds, there is no reduction to practice. These patents are further limited by the fact that in order to use the chymosin for the commercial production of cheese, chymosin would have to be purified from the seed or milled seeds.

PCT patent application WO 92/01042 discloses the expression of chymosin in the leaves of transgenic tobacco and potato plants. According to the disclosure chymosin expression levels of only 0.1% to 0.5% (w/w) of total soluble leaf protein were attained. The methodology of WO 92/01042 is further limited in that the production in leaves would require immediate extraction of the enzyme from the leaf material upon harvesting of the plants as the enzyme would lose activity when stored in leaves. In addition, due to the relatively high water content of leaves, large amounts of biomass must be processed.

WO 92/18634 discloses a promoter isolated from a seed-specific acyl protein (ACP) gene, capable of expressing a gene under the control of said promoter before or during fatty acid or lipid biosynthesis in plant cells.

US 5,714,474 discloses a method for the production of enzymes in seeds and their use.

There is a need in the art to further improve methods for the recombinant expression of chymosin in plants.

### SUMMARY OF THE INVENTION

The present invention relates to novel and improved methods of producing commercial levels of chymosin in transgenic plants. The inventors have found that chymosin when expressed in the seeds of transgenic plants accumulates to levels of at least 0.5% (w/w) of total seed protein.

Accordingly, the invention provides a method for the production of chymosin in a plant seed comprising:
a) introducing into a plant cell a chimeric nucleic acid sequence molecule comprising in the 5' to 3' direction of transcription:
   1) a first nucleic acid sequence capable of regulating transcription in said plant cell operatively linked to;
   2) a second nucleic acid sequence encoding a chymosin polypeptide operatively linked to;
   3) a third nucleic acid sequence capable of terminating transcription in said plant cell;
b) growing said plant cell into a mature plant capable of setting seed; and
c) obtaining seed from the mature plant wherein said seed contains chymosin; and
d) isolating said chymosin from seed, wherein isolating said chymosin from seed comprises:
   (i) crushing of the plant seed to obtain crushed plant seed;
   (ii) fractionating the crushed plant seed into an oil fraction, aqueous fraction and a fraction comprising insoluble material;
   (iii) contacting the aqueous fraction with a protein binding resin; and
   (iv) recovering the chymosin from the protein binding resin.

Preferably, at least 0.5% (w/w) of the total seed protein is chymosin.

The present invention also provides a method for the production of plant seeds containing at least 0.5% (w/w) chymosin in the total seed protein comprising:
(a) introducing into each of at least two plant cells a chimeric nucleic acid sequence molecule comprising in the 5' to 3' direction of transcription:
   1) a first nucleic acid sequence which is a seed-specific promoter and is capable of regulating transcription in said plant cell operatively linked to;
   2) a second nucleic acid sequence encoding a chymosin polypeptide operatively linked to;
   3) a third nucleic acid sequence capable of terminating transcription in said plant cell;
(b) growing each plant cell into a mature plant capable of setting seed;
(c) obtaining seed from each mature plant;
(d) detecting the levels of chymosin in the seed of each plant obtained in step (c) or in the seed of a plant generated from the seed of a plant obtained in step (c); and
(e) selecting plants that contain at least 0.5% (w/w) chymosin in the total seed protein.

The nucleic acid sequence capable of regulating transcription is a seed-specific promoter. In preferred methods, the chimeric nucleic acid sequence additionally comprises a signal sequence capable of targeting the chymosin polypeptide to the plant apoplast. In further preferred methods, the nucleic acid sequence encoding chymosin sequence is optimized for plant codon usage and the chymosin sequence further contains the chymosin pro-peptide or pre-pro-peptide or pre-peptide sequences.

In a further aspect, the present invention provides plant seeds comprising heterologously expressed chymosin wherein said heterologously expressed chymosin is at least 0.5% (w/w) of the total seed protein. In a preferred embodiment of the present invention, the plant seeds comprise a chimeric nucleic acid sequence comprising in the 5' to 3' direction of transcription:
1) a first nucleic acid sequence capable of regulating transcription in said plant cell operatively linked to;
2) a second nucleic acid sequence encoding a chymosin polypeptide operatively linked to;
3) a third nucleic acid sequence capable of terminating transcription in said plant cell wherein the seed contains chymosin.

In another aspect the present invention provides plants capable of setting seed expressing chymosin wherein said heterogeneously expressed chymosin is at least 0.5% (w/w) of the total seed protein. In a preferred embodiment of the invention, the plants capable of setting seed comprise a chimeric nucleic acid sequence comprising in the 5' to 3' direction of transcription:
1) a first nucleic acid sequence capable of regulating transcription in a plant cell operatively linked to;
2) a second nucleic acid sequence encoding a chymosin polypeptide operatively linked to;
3) a third nucleic acid sequence capable of terminating transcription in said plant cell, wherein the seed contains chymosin.
Isolation of chymosin from seed comprising:
(i) crushing of the plant seed to obtain crushed plant seed;
(ii) contacting the crushed plant seed or a fraction thereof with a protein binding resin; and
(iii) recovering the chymosin from the protein binding resin is taught herein for reference.

In a preferred embodiment the protein binding resin is a hydrophobic interaction resin. In further preferred embodiments of the invention, the isolation of the chymosin further comprises the employment of an ion exchange resin and a hydrophobic interaction resin.

### BRIEF DESCRIPTION OF THE DRAWINGS

The invention will now be described in relation to the drawings in which:
Figure 1 shows the nucleotide sequence (SEQ.ID.NO.:1) and corresponding amino acid sequence (SEQ.ID.NO.:2) of the open reading frame of a pre-pro-chymosin sequence. The "pre" sequence is indicated in Italics between and including amino acids 1 to 26. The "pre" sequence encodes a signal sequence identical to the PR-S signal sequence from tobacco sequence (Sijmons et al. (1990) Bio/technology 8: 217-221). Amino acids 27 to 67 inclusive are the "pro" sequence with the remaining amino acids encoding the mature chymosin polypeptide.
Figure 2 shows the nucleotide sequence (SEQ.ID.NO.:3) of the phaseolin promoter- a pre-pro-chymosin-phaseolin terminator sequence responsible for the high levels of expression of chymosin in plant seeds.
Figure 3 is a Western blot analysis comparing a chymosin standard and a protein extract of seeds from a *Brassica* plant expressing chymosin.
Figure 4 is a bar diagram showing the expression of chymosin in flax seeds derived from independent transformed flax plants.
Figure 5 shows a SDS-polyacrylamide gel showing progressive purification of chymosin obtained from transgenic seeds of *Brassica napus* as described in example 5.

### DETAILED DESCRIPTION OF THE INVENTION

As hereinbefore mentioned, the present invention relates to improved methods for the production of chymosin in transgenic plants. The present inventors have surprisingly found that by expressing chymosin in the seeds of plants, chymosin accumulation levels exceeding 0.5% (w/w) of total seed protein may be attained. These high expression levels in plant seeds allow significant commercial savings since the acreage of plants that needs to be grown can be limited and the amount of biomass that must to be subjected to extraction is reduced. The amount of biomass processed is further limited due to the relatively low water content present in plant seed. Furthermore, the expression in plants seed offers flexibility in storage and shipment of chymosin as a raw material, since chymosin retains its enzymatic activity upon extraction from stored seed.

Accordingly, the invention provides a method for producing chymosin in plant seeds comprising:
a) introducing into a plant cell a chimeric nucleic acid sequence molecule comprising in the 5' to 3' direction of transcription:
   1) a first nucleic acid sequence capable of regulating transcription in said plant cell operatively linked to;
   2) a second nucleic acid sequence encoding a chymosin polypeptide operatively linked to;
   3) a third nucleic acid sequence capable of terminating transcription in said plant cell;
b) growing said plant cell into a mature plant capable of setting seed; and
c) obtaining said seed from said mature plant wherein the seed contains chymosin, and
d) isolating said chymosin from seed, wherein isolating said chymosin from seed comprises:
   (i) crushing of the plant seed to obtain crushed plant seed;
   (ii) fractionating the crushed plant seed into an oil fraction, aqueous fraction and a fraction comprising insoluble material;
   (iii) contacting the aqueous fraction with a protein binding resin; and
   (iv) recovering the chymosin from the protein binding resin.

In a preferred embodiment, at least 0.5% (w/w) of the total seed protein is chymosin. More preferably at least 1% (w/w) of the total seed protein is chymosin, even more preferably at least 2% (w/w) of the total seed protein is chymosin and most preferably at least 4% (w/w) of the total seed protein is chymosin.

As used herein the term "chymosin polypeptide" refers to all chymosins and includes pre-pro-chymosin and pro-chymosin polypeptides. The chymosin is preferably mammalian such as bovine, goat and sheep chymosin.

The term "nucleic acid sequence encoding a chymosin polypeptide" refers to all nucleic acid sequence encoding chymosin and all nucleic acid sequences that hybridize thereto under stringent hybridization conditions or would hybridize thereto but for the use of synonymous codons.

Appropriate "stringent hybridization conditions" which promote DNA hybridization are known to those skilled in the art, or may be found in Current Protocols in Molecular Biology, John Wiley & Sons, N.Y. (1989), 6.3.1-6.3.6. For example, the following may be employed: 6.0 x sodium chloride/sodium citrate (SSC) at about 45°C, followed by a wash of 2.0 x SSC at 50°C. The stringency may be selected based on the conditions used in the wash step. For example, the salt concentration in the wash step can be selected from a high stringency of about 0.2 x SSC at 50°C. In addition, the temperature in the wash step can be at high stringency conditions, at about 65°C.

The term "nucleic acid sequence encoding a chymosin polypeptide" includes nucleic sequences that encode pre-pro-chymosin and pro-chymosin. In addition, the nucleic acid sequences that encode chymosin may be linked to additional nucleic acid sequences such as those that encode signal peptides.

In preferred embodiments of the present invention, nucleic acid sequences encoding bovine chymosin A or chymosin B are used (Moir et al. (1982) Gene 19: 127-138.; Harris et al. (1982) Nucleic Acids Res. 10: 2177-2187). In further preferred embodiments nucleic acid sequences encoding chymosin are used which have been optimized for codon usage in plants. The natural bovine chymosin sequence has a GC content of 56% with a preference for a G or C in the third position of the codon. This differs from the codon bias for cattle which has an average GC content of 39% (Mishimori et al. (1982) J Biochem 91: 1085-1088). In a preferred embodiment, the codon usage of chymosin is manipulated to reflect a codon usage typical of seed-storage proteins found in oilseeds, for example using a GC content of 49% with a preference for a G or C in the third position of the codon (see Example 1).

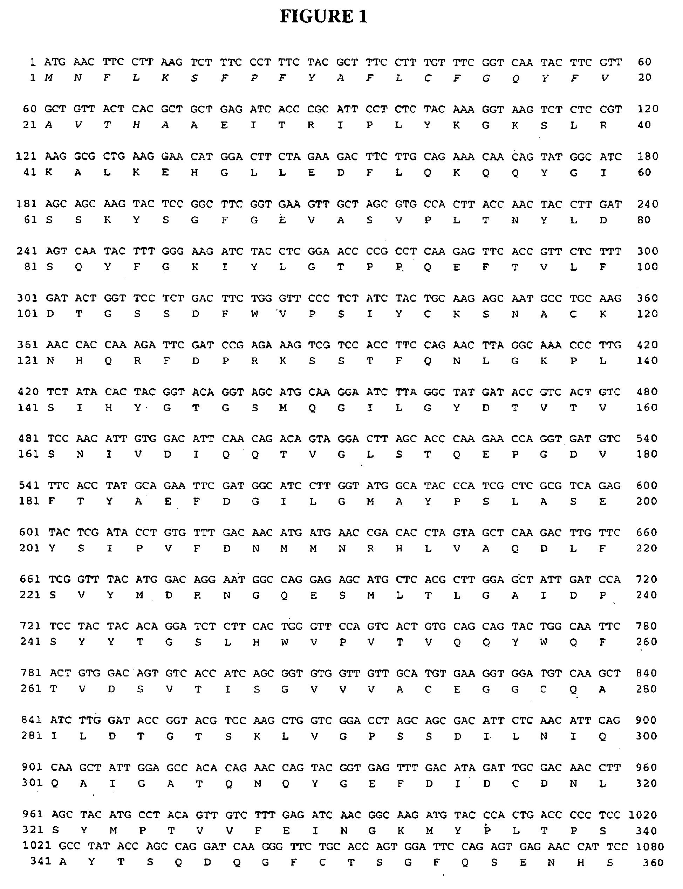

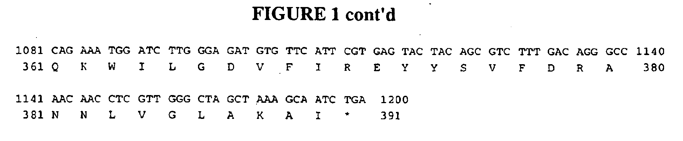

The invention further includes the use of nucleic acid sequences encoding chymosin precursor proteins that can be activated, for example by treating the precursor polypeptide at low pH, to exhibit chymosin activity. Nucleic acid sequences encoding chymosin precursor proteins that may be used in accordance with the present invention include naturally occurring nucleic acid sequences encoding chymosin precursor proteins, such as "pro-chymosin", "pre-chymosin" and "pre-pro-chymosin", as well as non-naturally occurring nucleic acid sequences encoding precursor proteins comprising chymosin and capable of activation to exhibit chymosin activity. In a preferred embodiment of the invention, a nucleic acid sequence encoding bovine pro-chymosin comprising 42 extra amino acid residues is used (Moir et al. (1982) Gene 19: 127-138.; Harris et al. (1982) Nucleic Acids Res.10: 2177-2187). Other nucleic acid sequences encoding precursor proteins that may be used in accordance with the present invention include those encoding bovine pre-pro-chymosin comprising 58 extra amino acid residues (Moir et al. (1982) Gene 19: 127-138.; Harris et al. (1982) Nucleic Acids Res.10: 2177-2187), and nucleic acid sequences encoding plant signal sequences capable of targeting chymosin to a preferred subcellular compartment, for example the plant apoplast, the golgi apparatus or cytoplasm. In one preferred embodiment, the nucleic acid sequence encoding chymosin comprises a nucleic acid sequence encoding the tobacco pathogenesis related protein-S (PR-S) signal sequence (Sijmons et al. (1990) Bio/technology 8: 217-221.) directing targeting to the plant apoplast linked to a nucleic acid sequence encoding a bovine pro-chymosin polypeptide sequence (Figure 1 and SEQ.ID.1). Other naturally occurring signal sequences that could be used in accordance with the present invention include for example the barley alpha amylase signal sequence (Rogers (1985) J. Biol. Chem. 260(6): 3731-3738) directing targeting of the chymosin sequence to the apoplast. The nucleic acid sequences encoding additional peptide sequences may be homologous as well as heterologous with respect to the nucleic acid sequence encoding the chymosin polypeptide. The nucleic acid sequence encoding the additional peptide sequences, such as the pro-peptide, pre-pro-peptide or pre-peptide, may vary in length and are preferably codon-optimized for use in plants.

In embodiments of the invention involving the activation of a chymosin precursor protein, the activation reaction may be performed upon obtaining the plant seeds by for example treating an extracted seed fraction at low pH, preferably at pH values lower than 5, or the activation reaction may take place in *planta*. It is also possible to complete the activation reaction in a mixture comprising chymosin precursor polypeptides and enzymatically active chymosin. The chymosin precursor protein may be partially active or exhibiting no chymosin activity, however the precursor protein is typically not fully active.

Nucleic acid sequences encoding chymosin are readily available or obtainable by the skilled artisan based on chymosin nucleic acid sequences and/or amino acid sequences known in the art. The bovine nucleic acid and amino acid sequences for chymosin A and chymosin B for example, are known and may be directly used in accordance with the present invention. As well, the complete primary structure of lamb preprochymosin has been deduced from cDNA (Pungercar et al. (1990) Nucleic Acids Res. 18(15): 4602). These known chymosin nucleic acid sequences may also be used to design and construct probes to identify previously undiscovered nucleic acid sequences encoding chymosin. These probes may be used to isolate nucleic acid sequence encoding chymosin from for example cDNA or genomic libraries. The nucleic acid sequence encoding chymosin is preferably obtained from a mammal. Thus additional nucleic acid sequence chymosin sequences may be discovered and used in accordance with the present invention.

The term "nucleic acid sequence" as used herein refers to a sequence of nucleotide or nucleoside monomers consisting of naturally occurring bases, sugars and intersugar (backbone) linkages. The term also includes modified or substituted sequences comprising non-naturally occurring monomers or portions thereof, which function similarly. The nucleic acid sequences of the present invention may be ribonucleic (RNA) or deoxyribonucleic acids (DNA) and may contain naturally occurring bases including adenine, guanine, cytosine, thymidine and uracil. The sequences may also contain modified bases such as xanthine, hypoxanthine, 2-aminoadenine, 6-methyl, 2-propyl, and other alkyl adenines, 5-halo uracil, 5-halo cytosine, 6-aza uracil, 6-aza cytosine and 6-aza thymine, pseudo uracil, 4-thiouracil, 8-halo adenine, 8-amino adenine, 8-thiol adenine, 8-thio-alkyl adenines, 8-hydroxyl adenine and other 8-substituted adenines, 8-halo guanines, 8-amino guanine, 8-thiol guanine, 8-thioalkyl guanines, 8-hydroxyl guanine and other 8-substituted guanines, other aza and deaza uracils, thymidines, cytosines, adenines, or guanines, 5-trifluoromethyl uracil and 5-trifluoro cytosine.

In accordance with the present invention, the chimeric nucleic acid sequences can be incorporated in a known manner in a recombinant expression vector which ensures good expression in a plant seed. Accordingly, the present invention includes a recombinant expression vector comprising a chimeric nucleic acid sequence of the present invention suitable for expression in a seed cell.

The term "suitable for expression in a seed cell" means that the recombinant expression vectors contain the chimeric nucleic acids sequence of the invention, a regulatory region and a termination region, selected on the basis of the seed cell to be used for expression, which is operatively linked to the nucleic acid sequence encoding the polypeptide of desirable amino acid composition. Operatively linked is intended to mean that the chimeric nucleic acid sequence encoding the polypeptide is linked to a regulatory sequence and termination region which allows expression in the seed cell. A typical construct consists, in the 5' to 3' direction of a regulatory region complete with a promoter capable of directing expression in a plant, a chymosin coding region and a transcription termination region functional in plant cells. These constructs may be prepared in accordance with methodology well known to those of skill in the art of molecular biology (see for example: Sambrook et al. (1990) Molecular Cloning, 2nd ed. Cold Spring Harbor Press). The preparation of constructs may involve techniques such as restriction digestion, ligation, gel electrophoresis, DNA sequencing and PCR. A wide variety of cloning vectors are available to perform the necessary cloning steps. Especially suitable for this purpose are the cloning vectors with a replication system that is functional in *Escherichia coli* such as pBR322, the pUC series M13mp series, pACYC184, pBluescript etc. The nucleic acid sequence may be introduced into these vectors and the vectors may be used to transform E. coli which may be grown in an appropriate medium. Plasmids may be recovered from the cells upon harvesting and lysing the cells. Final constructs may be introduced into plant vectors compatible with integration into the plant such as the Ti and Ri plasmids.

The selection of regulatory sequences will determine the plant organ in which the protein is expressed and may influence the level that a gene will be transcribed. Regulatory sequences are art-recognized and are selected to direct expression in the plant cell. Accordingly, the term "regulatory sequence" includes promoters, enhancers, ribosome binding sites, introns and other expression elements. Examples of promoters include both non-seed specific, constitutive promoters such as the 35-S CaMV promoter (Rothstein et al. (1987) Gene 53: 153-161) and seed specific promoters such as the phaseolin promoter (Sengupta-Gopalan et al., (1985) PNAS USA 82: 3320-3324) or the *Arabidopsis* 18 kDa oleosin promoter (van Rooijen et al., (1992) Plant Mol. Biol. 18: 1177-1179). In preferred embodiments of the present invention, seed specific promoters are employed and more specifically the phaseolin promoter. Enhancers which may be used include the AMV leader (Jobling and Gehrke (1987) Nature 325: 622-625) to increase the expression levels. It should be understood that the design of the expression vector may depend on such factors as the choice of the plant species and /or the type of polypeptide to be expressed.

The region containing the transcriptional terminator sequence preferably includes from about 200 to about 1,000 nucleotide base pairs and may comprise any such sequences functional in plants, such as the nopaline synthase termination region (Bevan et al., (1983) Nucl. Acid. Res. 11: 369-385), the phaseolin terminator (Van der Geest et al., (1994) Plant J. 6(3): 413-423), the terminator for the octopine synthase gene of *Agrobacterium tumefaciens* or other similarly functioning elements. These transcription terminator regions can be obtained as described by An (1987) Methods in Enzym. 153: 292 or are already present in plasmids available from commercial sources such as ClonTech, Palo Alto, California. The choice of the appropriate terminator may have an effect of the rate of transcription. In preferred embodiments of the invention the phaseolin terminator is employed.

The expression vectors may also contain a marker gene. Marker genes comprise all genes that enable distinction of transformed plant cells from non-transformed cells, including selectable and screenable marker genes. Conveniently, a marker may be a resistance marker to a herbicide, for example, glyphosate or phosphinothricin, or to an antibiotic such as kanamycin, G418, bleomycin, hygromycin, chloramphenicol and the like, which confer a trait that can be selected for by chemical means. Resistance markers to a herbicide when linked in close proximity to the chymosin gene may be used to maintain selection pressure on a population of transgenic plants for those plants that have not lost the gene of interest. Screenable markers may be employed to identify transformants through observation. They include but are not limited to the beta-glucuronidase or *uidA* gene, a beta-lactamase gene or a green fluorescent protein (Niedz et al. (1995) Plant Cell Rep. 14: 403).

A variety of techniques are available for the introduction of nucleic acid sequences, in particular DNA, into plant host cells. For example, the chimeric DNA constructs may be introduced into host cells obtained from dicotelydenous plants, such as tobacco, and oleagenous species, such as Brassica napus using standard *Agrobacterium* vectors by a transformation protocol such as described by Moloney et al. (1989) Plant Cell Rep. 8: 238-242 or Hinchee et al. (1988) Bio/Technol. 6: 915-922; or other techniques known to those skilled in the art. For example, the use of T-DNA for transformation of plant cells has received extensive study and is amply described in EP 0 120 516, Hoekema et al., (1985), Chapter V In: The Binary Plant Vector System Offset-drukkerij Kanters BV, Alblasserdam); Knauf et al. (1983), Genetic Analysis of Host Expression by Agrobacterium, p. 245, In: Molecular Genetics of Bacteria-Plant Interaction, Puhler, A. ed. Springer-Verlag, NY); and An et al., (1985) EMBO J., 4: 277-284. *Agrobacterium* transformation may also be used to transform monocot plant species (US Patent 5,591,616).

Conveniently, explants may be cultivated with *Agrobacterium tumefaciens* or *Agrobacterium rhizogenes* to allow for the transfer of the transcription construct in the plant host cell. Following transformation using *Agrobacterium* the plant cells are dispersed into an appropriate medium for selection, subsequently callus, shoots and eventually plants are recovered. The *Agrobacterium* host will harbour a plasmid comprising the *vir* genes necessary for transfer of the T-DNA to plant cells. For injection and electroporation (see below) disarmed Ti-plasmids (lacking the tumour genes, particularly the T-DNA region) may be introduced into the plant cell.

The use of non-*Agrobacterium* techniques permits the use of constructs described herein to obtain transformation and expression in a wide variety of monocotyledenous and dicotelydenous plant species. These techniques are especially useful for transformation of plant species that are intractable in an *Agrobacterium* transformation system. Other techniques for gene transfer include particle bombardment (Sanford, (1988) Trends in Biotechn. 6: 299-302), electroporation (Fromm et al., (1985) PNAS USA, 82: 5824-5828; Riggs and Bates, (1986) PNAS USA 83: 5602-5606), PEG mediated DNA uptake (Potrykus et al., (1985) Mol. Gen. Genetics., 199: 169-177), microinjection (Reich et al., Bio/Techn. (1986) 4:1001-1004) and silicone carbide whiskers (Kaeppler et al. (1990) Plant Cell Rep. 9: 415-418).

In a specific application such as to B. *napus,* the host cells targeted to receive recombinant DNA constructs typically will be derived from cotyledonary petioles as described by Moloney et al. (1989) Plant Cell Rep. 8: 238-242. Other examples using commercial oil seeds include cotyledon transformation in soybean explants (Hinchee et al., (1988) Bio/Technol. 6: 915-922 and stem transformation of cotton (Umbeck et al., (1987) Bio/Technol. 5: 263-266).

Following transformation, the cells, for example as leaf discs, are grown in selective medium. Once the shoots begin to emerge, they are excised and placed onto rooting medium. After sufficient roots have formed, the plants are transferred to soil. Putative transformed plants are then tested for presence of a marker. Southern blotting may be performed on genomic DNA using an appropriate probe, to show integration into the genome of the host cell.

Transformed plants grown in accordance with conventional agricultural practices, are allowed to set seed. See, for example, McCormick et al. (1986) Plant Cell Reports 5: 81-84. The chymosin expression level that is attained in accordance with the present invention, is generally expected to vary somewhat depending on the transformed plant that is assayed. As hereinbefore mentioned for the process to be economically attractive, a minimum expression level is required. The terms "commercial" and "commercial levels" as used herein denote an expression level wherein at least 0.5% (w/w) and more preferably more than 2% (w/w) and most preferably more than 4% (w/w) of total seed protein is chymosin. Preferably expression levels are determined using quantitative Western blotting using the methodology described in detail in Example 2. Accordingly, typically a variety of transformed plants are screened and the expression level of chymosin in seed is determined. It is expected that typically between 5 and 50 plants may need to be screened to identify at least one plant expressing commercial levels of chymosin. Seeds obtained from plants expressing commercial levels of chymosin (i.e. at least 0.5% (w/w) of the total seed protein) are selected for further propagation.

Accordingly, the present invention provides a method for the production of plant seeds containing at least 0.5% (w/ w) chymosin in the total seed protein comprising:
(a) introducing into each of at least two plant cells a chimeric nucleic acid sequence molecule comprising in the 5' to 3' direction of transcription:
   1) a first nucleic acid sequence which is a seed-specific promoter and is capable of regulating transcription in said plant cell operatively linked to;
   2) a second nucleic acid sequence encoding a chymosin polypeptide operatively linked to;
   3) a third nucleic acid sequence capable of terminating transcription in said plant cell;
(b) growing each plant cell into a mature plant capable of setting seed;
(c) obtaining seed from each mature plant;
(d) detecting the levels of chymosin in the seed of each plant obtained in step (c) or in the seed of a plant generated from the seed of a plant obtained in step (c); and
(e) selecting plants that contain at least 0.5% (w/w) chymosin in the total seed protein.

Chymosin activity can be assayed by spectrophotometric or fluorometric methods or by milk-clotting assays. In the milk-clotting assay, a diluted sample is added to a milk solution so that the final solution contains 8% skim milk and 0.05% CaCl₂ in water. The clotting time or flake point is measured as the time it takes for the thin film of milk to break into visible particles. The concentration of chymosin is determined by comparing to a linear standard plotted as clotting time in seconds against the chymosin concentration (Tsuchiya et al. (1993) AppL Microbiol. Biotechnol. 40: 327-332).

Two or more generations of plants may be grown and either crossed or selfed to allow identification of plants and strains with desired phenotypic characteristics including production of the recombinant polypeptide. It may be desirable to ensure homozygosity in the plants to assure continued inheritance of the recombinant trait Methods for selecting homozygous plants are well known to those skilled in the art of plant breeding and include recurrent selfing and selection and anther and mircospore culture. Homozygous plants may also be obtained by transformation of haploid cells or tissues followed by regeneration of haploid plantlets subsequently converted to diploid plants by any number of known means (e.g. treatment with colchicine or other microtubule disrupting agents).

The present invention also provides plant seeds comprising heterologously expressed chymosin wherein said heterologously expressed chymosin is at least 0.5% (w/w) of the total seed protein expressing chymosin. In a preferred embodiment of the present invention the plant seeds comprise a chimeric nucleic acid sequence comprising in the 5' to 3' direction of transcription:
1) a first nucleic acid sequence capable of regulating transcription in said plant cell operatively linked to;
2) a second nucleic acid sequence encoding a chymosin polypeptide operatively linked to;
3) a third nucleic acid sequence capable of terminating transcription in said plant cell, wherein the seed contains chymosin.

In a further aspect the present invention provides plants capable of setting seed expressing chymosin, wherein said heterogeneously expressed chymosin is at least 0.5% (w/w) of the total seed protein. In a preferred embodiment of the invention, the plants capable of setting seed comprise a chimeric nucleic acid sequence comprising in the 5' to 3' direction of transcription:
1) a first nucleic acid sequence capable of regulating transcription in said plant cell operatively linked to;
2) a second nucleic acid sequence encoding a chymosin polypeptide operatively linked to;
3) a third nucleic acid sequence capable of terminating transcription in said plant cell, wherein the seed contains chymosin.

The methods disclosed in the present invention can be used over a broad range of plant species. Particularly preferred plant cells employed in accordance with the present invention include cells from the following plants: soybean (*Glycine max),* rapeseed (*Brassica napus, Brassica campestris*), sunflower (*Helianthus annuus*), cotton (*Gossypium hirsutum*), corn *(Zea mays),* tobacco (*Nicotiana tabacum*), alfalafa *(Medicago sativa),* wheat *(Triticum sp.),* barley (*Hordeum vulgare*), oats (*Avena sativa* L.), sorghum (*Sorghum bicolor*), Arabidopsis thaliana, potato (*Solanum sp*.), flax/linseed *(Linum usitatissimum),* safflower (*Carthamus tinctorius*), oil palm *(Elaeis guineensis),* groundnut (*Arachis hypogaea*), Brazil nut *(Bertholletia excelsa)* coconut *(Cocos nucifera),* castor *(Ricinus communis),* coriander (*Coriandrum sativum*), squash (*Cucurbita maxima*), jojoba *(Simmondsia chinensis*) and rice (*Oryza sativa).*
The term "crushing" as used herein refers to any process or methodology to comminute seed and includes mechanical pressing, grinding, crushing processes and the like. Preferably the seeds are ground using a mill such as for example a colloid mill, a disk mill, a pin mill, an orbital mill, an IKA mill, a homogenizer or similar equipment. The selection of the crushing equipment depends inter alia on the throughput requirements and on the seed source. Typically the crushing conditions selected result in the breakage of individual seed cells. It is of importance however that the chymosin polypeptide remains intact. Crushing conditions that would substantially inactivate the enzyme are undesirable in the practice of the present invention. The crushing process practiced in accordance with the present invention permits the recovery of a crushed plants seeds comprising chymosin.

The crushing process may be carried out using dry seed. Preferably however the seeds are crushed in the presence of water or a buffer. Prior to, during or after the crushing process, additional water or a buffer may be employed to dilute the seed extract. Preferably the crushed seed fraction obtained is between 2 and 100 fold diluted relative to the original seed volume. Furthermore the salt concentration may be adjusted by the addition of extraneous salts or salt solutions to the crushed seeds. Accordingly, preferably the extraneous salt concentration of the crushed seed that is obtained is preferably between approximately 0.1M and 2M. Suitable salts to adjust the salt concentration in accordance with the present invention include sulfate salts for example sodium sulfate, magnesium sulfate, and ammonium sulfate; phosphate salts, for example sodium phosphate, magnesium phosphate and ammonium phosphate; chloride salts, for example sodium chloride and calcium chloride; and mixtures thereof. A preferred salt used in accordance with the present invention is sodium chloride.

Upon crushing of the seed it is generally preferable to prepare an aqueous fraction of the crushed plant seeds by the removal of the insoluble material and the oil fraction of the seed. The insoluble material is substantially insoluble or in an insolublized association with insoluble material produced upon crushing of the plant seed material. The insoluble material is either produced in the plant seed or may be associated with the plant seed in the form of insoluble aggregates including, seed hulls, fibrous material, carbohydrates or external contaminants such as soil particles and the like. The process permits the separation of soluble seed material from insoluble seed material. Any suitable methodology may use be accomplished using any methodology that allows the separation of the seed insoluble material from the soluble seed constituents, including for example gravitation based methods such as for example centrifugation or size exclusion based methods such as filtration. In a preferred embodiment of the present invention centrifugation is used. Centrifugation equipment that may be used in accordance with the present invention includes a tubular bowl centrifuge, a decantation centrifuge, a hydrocyclone, a disk stack centrifuge, and the like.

Removal of the oil fraction is particularly desirable when chymosin is produced in seeds comprising a relatively high oil content such as rapeseed, flax, sunflower seed and the like. Any suitable methodology may be used that allows the separation of the oil fraction from the aqueous fraction of the seed, including for example gravitation based methods such as for example centrifugation or size exclusion based methods such as filtration. In a preferred embodiment of the present invention centrifugation is used. Centrifugation equipment that may be used in accordance with the present invention includes a tubular bowl centrifuge, a decantation centrifuge, a hydrocyclone, a disk stack centrifuge, and the like.

Generally the solids are removed prior to the oil fraction, however in other embodiments of the invention the removal of insoluble seed constituents and the oil fraction is accomplished concomitantly using a gravity based method such as a 3-phase tubular bowl centrifuge or decanter or a size-exclusion based separation method.

In a further preferred embodiment selective precipitation of the crushed plant seed extract or fraction thereof may be performed prior to contacting the plant seed extract or fraction thereof with the protein binding resin. This selective precipitation step is preferably accomplished by selecting any conditions that allow the precipitation of at least 50% (w/w) of the endogenous seed proteins while substantially all chymosin remains soluble. With the term "substantially all" it is meant that at least approximately 75% (w/w) of all chymosin remains soluble. In a more preferred embodiment at least 85% (w/w) of all chymosin remains soluble. In the most preferred embodiment at least approximately 90% (w/w) of all chymosin remains soluble. In preferred embodiments of the present invention precipitation is accomplished by adjusting the pH of the crushed seed extract. The pH of the crushed seed is preferably adjusted to a pH of less than approximately 5.5. More preferably the pH is adjusted to a pH of between approximately 1.5 and 3.5. Most preferably the pH is adjusted to a pH of approximately 2.0. Any suitable acid my be used to adjust the pH, such as hydrochloric acid, sulfuric acid, phosphoric acid and the like preferably having a pH of less than 2. The precipitation step may take place concommitantly with the crushing step. In preferred embodiments, the precipitation step is performed subsequent to the seed-crushing step. Furthermore the precipitation may be performed prior to or subsequent to either the removal of the insoluble material or removal of the oil fraction. It is preferred however to remove the insoluble material and the oil fraction prior to selective precipitation.

The term "protein binding resin" means any resin that is capable of binding to proteins, in particular chymosin. In a preferred embodiment, the protein binding resin is a hydrophobic interaction resin.

The present inventors have found that a hydrophobic interaction resin is particularly useful in isolating chymosin from plant seeds. A "hydrophobic interaction resin" refers to any protein compatible resin capable of differentially binding proteins present in a mixture of proteins, said differential binding occurring as a result of differences in hydrophobic characteristics of the proteins present in the mixture. Hydrophobic interaction resins are generally art-recognized and include for example sepharose resins having functional groups such as alkyl groups (e.g. butyl-sepharose, octyl-sepharose) and phenyl groups (e.g. phenyl-sepharose) and superose resins having functional groups such as alkyl groups and phenyl groups. The hydrophobic interaction resin may be used batch-wise or prepared for column chromatography.

In the practice of the present invention the crushed seed extract or a fraction thereof comprising chymosin is contacted with the hydrophobic interaction resin under conditions that will permit chymosin to bind to the hydrophobic interaction resin. Preferred binding conditions in accordance with the present invention are conditions of high ionic strength, for example 1M to 2M salt concentrations, e.g. 1.5M ammonium sulphate. Other salts that may be used in accordance with the present invention include sulfate salts for example magnesium sulfate; phosphate salts, for example sodium phosphate, magnesium phosphate and ammonium phosphate; chloride salts, for example sodium chloride and calcium chloride; and mixtures thereof. Once binding has been accomplished conditions are altered so that the bound substances are eluted differentially thus allowing the recovery of chymosin from the hydrophobic interaction column. Preferably the ionic strength is altered to accomplish elution, for example the ionic strength is reduced from 1.5 M to 0.5 M. The changes in conditions may be performed stepwise or gradually. Other elution methodologies that may be employed include reducing the eluent polarity for example using a glycol gradient up to 50%, adding chaotropic species such as urea, guanidine hydrochloride; the addition of detergents; changing pH or temperature.

In further preferred embodiments, chymosin is additionally purified by employing an ion exchange resin. An "ion exchange resin" refers to any protein compatible resinous material which is capable of binding charged compounds. Ion exchange columns are art recognized and include anion and cation exchange resins. These resins may be employed in a batch fashion or as a column. Preferred cation exchange columns for use in the present invention, include for example Pharmacia SP-Spehadex, Indion SP-2, IBF SP-Triacryl, IBF SP-Spherodex and the like. Preferred anion exchange resins in this regard are DEAE cellulose, IBF Q Spherodex, Pharmacia Q-Sephadex, Indion Q-2, IBF Q-Trisacryl and the like. In the practice of the present invention the aqueous solution of comprising chymosin is contacted with the ion-exchange resin under conditions at which the chymosin will bind to the resin. Whether chymosin binds to the resin depends on the pH of the aqueous solution, i.e. whether the pH is below or above the isoelectric point of chymosin (approximately 4.6). Accordingly, contacting the aqueous solution comprising chymosin under conditions at which chymosin will bind to the column refers to adjusting the pH of the solution above or below its isoelectric point so that it will bind to the selected resin. Binding of chymosin to the resin further depends on the ionic strength. Accordingly, the salt concentration may vary, for example a concentration of less than 250mM NaCl may be used. In order to elute chymosin of the resin conditions are selected which permit the elution of chymosin from the resin, preferably the ion concentration is adjusted to elute the chymosin of the resin. For example the salt concentration may be adjusted to a concentration of 2M NaCl. The pH and salt concentration of the chymosin preparation thus recovered may be adjusted as desired. The ion exchange resin step may be employed either prior or after the hydrophobic interaction step.

Optionally the chymosin preparation may be concentrated using for example ultrafiltration or treated for longer-term preservation using any suitable preservation methodology. For example the chymosin preparation may be sterilized using methodologies such as filtration or ultrafiltration.

Optionally the chymosin preparation may be concentrated using for example ultrafiltration or treated for longer-term preservation using any suitable preservation methodology. For example the chymosin preparation may be sterilized using methodologies such as filtration or ultrafiltration.

The following non-limiting examples are illustrative of the present invention:

### EXAMPLES

### EXAMPLE 1

### Construction of a plant transformation vector comprising of a chimeric nucleic acid sequence containing pre-pro-chymosin.

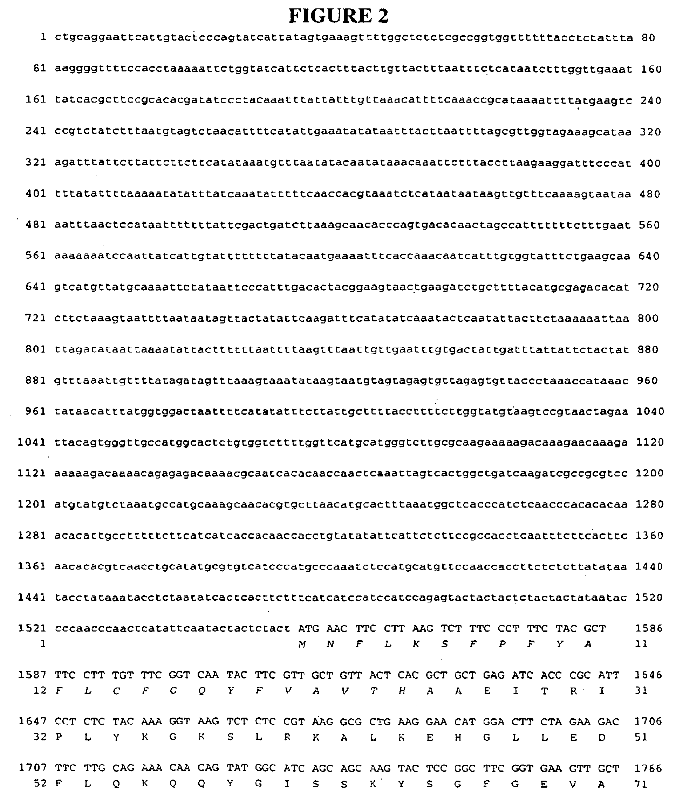

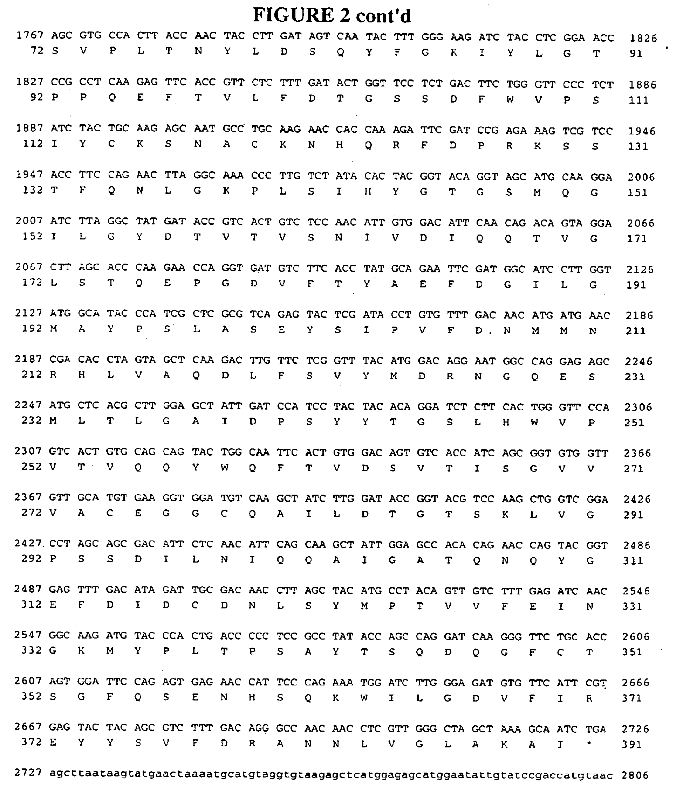

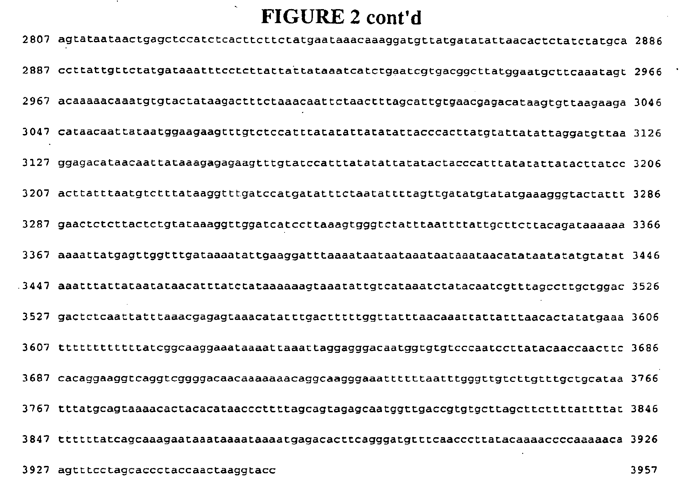

A pro-chymosin gene was re-synthesized from the bovine pro-chymosin to reflect the plant-preferred codons (See Figure 1 and SEQ.ID.NOS.: 1 and 2). Amino acids 27 to 67 are the pro-peptide sequence and amino acids 68 to 390 are the mature chymosin polypeptide. A PR-S signal sequence was attached to the 5' end of the pro-chymosin gene by PCR fusion. The PRS sequence includes amino acids 1 to 26 in Figure 1. The pre-pro-chymosin DNA fragment was fused in between a phaseolin promoter and the phaseolin terminator derived from the common bean *Phaseolus vulgaris* Slightom et al (1983) Proc. Natl Acad Sc USA 80: 1897-1901). A complete sequence of the phaseolin promoter-preprochymosin-phaseolin terminator insert responsible for the expression of chymosin in plant seeds is shown in Figure 2 and SEQ.ID.NO.:3. This insert was cloned into the PstI-KpnI sites of vector pSBS2004 and pSBS3000 and resulted in plasmids pSBS2151 and pSBS2165 respectively. pSBS2004 is a derivative from the *Agrobacterium* binary plasmid pCGN1559 (MacBride and Summerfield, 1990, Plant Molec. Biol. 14 269-276) in which, the CAMV 35S promoter-neomycin phosphotransferase gene- tumor morphology large locus 3' antibiotic selection cassette of pCGN1559 was replaced with parsley ubiquitin promoter-phosphinothricin acyltransferase gene-parsley ubiquitin termination sequence to confer resistance to the herbicide glufosinate ammonium. pSBS3000 is a derivative from the *Agrobacterium* binary plasmid pPZP221 (Hajdukiewicz et al., 1994, Plant Molec. Biol. 25: 989-994). In pSBS3000, the CaMV35S promoter-gentamycin resistance gene-CAMV 35S terminator of pPZP221 was replaced with parsley ubiquitin promoter-phosphinothricin acetyl transferase gene-parsley ubiquitin termination sequence to confer resistance to the herbicide glufosinate ammonium.

### EXAMPLE 2

### Generation of chymosin-expressing transgenic plants

Plasmids pSBS2151 and pSBS2165 were electroporated into Agrobacterium strain EHA101 (Hood, et al (1986) J Bacteriol 144: 732-743). Agrobacterium strain EHA101 (pSBS2151) was used to transform *Brassica napus*. The procedure for the transformation of *Brassica* has been essentially outlined in Moloney et al. (1989) Plant Cell Reports 8: 238-242, except phophinothricin, at a concentration of 1 to 2 mg/L, was used as the selectable agent. Agrobacterium strain EHA101 (pSBS2165) was used to transform flax cv Mc Gregor. Flax transformation was performed essentially as described in Jordan and McHughen (1988) Plant cell reports 7: 281-284, except transgenic shoots were selected on 10 µM L-phosphinothricine instead of kanamycin.

### EXAMPLE 3

### Expression levels of chymosin in Brassica

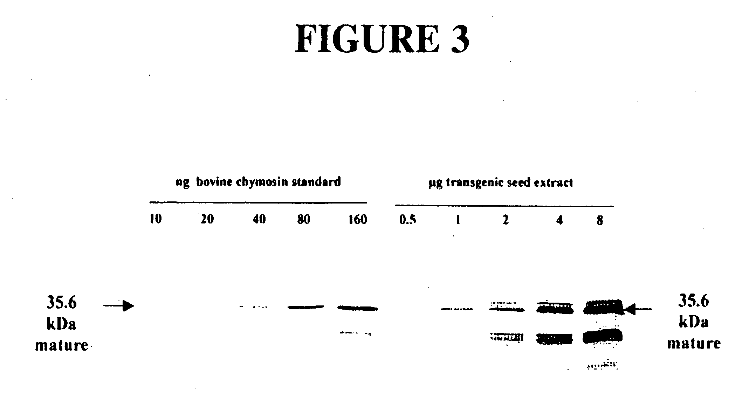

Physical characteristics of Brassica napus seed extracted chymosin were compared relative to commercially available bovine chymosin. The molecular weight of the two chymosin proteins was determined by gel electrophoresis on a 12% poly-acrylamide gel and Western blot analysis using a polyclonal rabbit antibody as shown in Figure 3. Specified concentrations were loaded onto a 12% poly-acrylamide gel and transferred to a membrane. The membrane was probed with a polyclonal antibody raised against commercial available bovine chymosin and visualized using alkaline phosphatase. This polyclonal antibody is immunologically reactive with several bands in the transgenic seed extract. Bands of the same electroforetic mobility are found in the commercial bovine chymosin extract. This suggests that the majority of the pre-pro-chymosin in the seed extract has matured into chymosin. The lower molecular weight bands likely result from proteolytic digestion of the mature protein and the minor higher molecular weight bands could correspond to altered processed forms of either preprochymosin or prochymosin. The protein levels for chymosin in one of the Brassica plants analyzed is shown in Figure 3. Seeds were ground in water to make a seed extract and the protein concentration was determined as described in Bradford (1976) Anal. Biochem. 72: 248-254. Different concentrations of the same seed extract were electrophoresed on a gel along with a bovine derived chymosin standard loaded with known concentrations. Western blot analysis was performed with a polyclonal rabbit antibody and visualized using alkaline phosphatase. Quantitative densitometry was used to correlate the density of the 35.6 kDa band to the concentration of the protein by comparison with a standard curve derived from known concentrations of chymosin. Table 1 is a compilation of the data for the amount of chymosin in the identical seed extract of differing concentrations and resulting percent of expression. The slightly different levels reflect a standard error. Note that no data is provided for 4 µg and 8 µg of seed extract as the results exceeded the saturation range of the densitometer.

The biological activity of the plant (Brassica) derived chymosin was determined through the use of milk-clotting assays. In the milk-clotting assay, a diluted seed extract sample is added to a clotting substrate as described in (Tsuchiya et al. (1993) Appl. Microbiol. Biotechnol. 40: 327-332). Transgenic Brassica seeds had the ability to clot milk whereas, seeds that were not transformed with the pro-chymosin gene were unable to clot milk.

### EXAMPLE 4

### Expression levels of chymosin in flax (Linum usitatissimum)

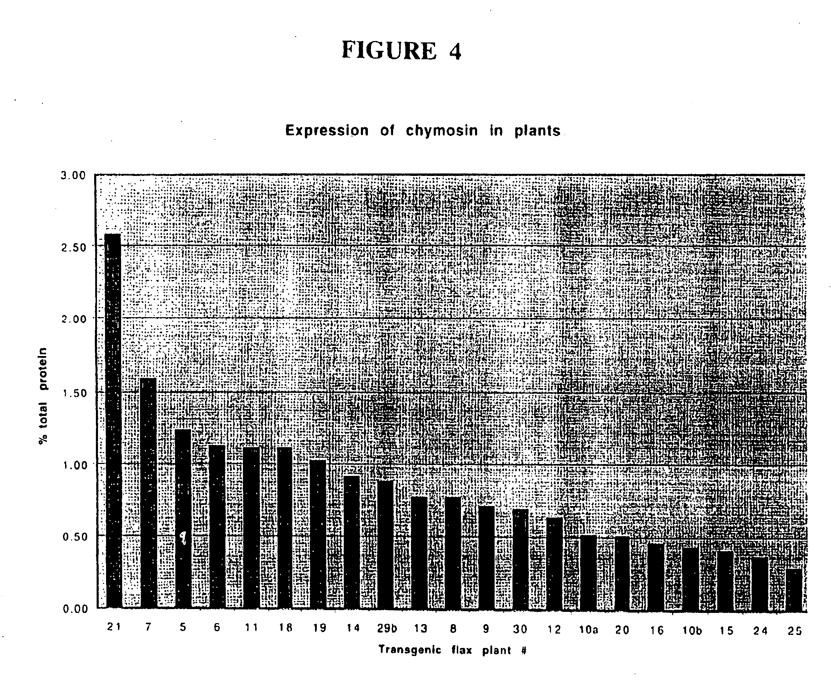

Transgenic flax plants containing the preprochymosin gene were analyzed for the presence of biologically active chymosin. The biological activity of the plant derived chymosin was determined through the use of milk-clotting assays. In the milk-clotting assay, a diluted flax seed extract sample is added to a clotting substrate as described in (Tsuchiya et al. (1993) Appl. Microbiol. Biotechnol. 40: 327-332). The clotting time or flake point is measured as the time it takes for the thin film of milk to break into visible particles. The concentration of chymosin in the seed extract is determined by comparing it to a linear standard curve plotted as clotting time in seconds against the chymosin concentration (Tsuchiya et al. (1993) Appl. Microbiol. Biotechnol. 40: 327-332). The chymosin concentration was first determined as a weight percentage of seed weight (=W%). The percentage chymosin as a percentage of total seed protein was calculated by using the formula (W/ percentage protein in dry seed) X 100. For flax the total amount of protein as a percentage of seed weight equals approximately 20 % (Gill, 1987, Linseed, Indian Council of Agricultural Research Publication). Wx5 equals the expression level of chymosin as a percentage of total seed protein. Figure 4 shows the expression levels of chymosin in transgenic flax seeds as a percentage of total protein for selected transformants.

### EXAMPLE 5

### Purification of chymosin from transgenic Brassica napus seed

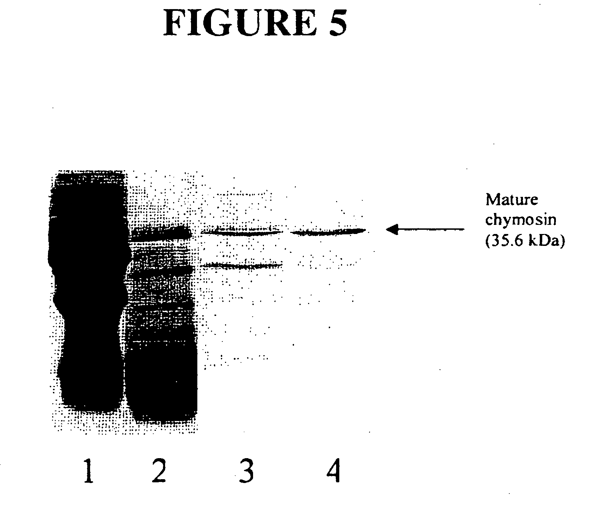

This example describes the laboratory-scale purification of chymosin from transgenic seed produced as described in example 2. Forty grams of transgenic *Brassica napus* seed containing recombinant chymosin was combined with 400 mls of a solution containing 250 mM NaCl. The mixture was ground using a polytron to produce a slurry releasing the chymosin into solution. This slurry was then centrifuged at approximately 10,000 x g to separate it into three phases, a solid pellet phase of insoluble material, an upper phase of seed oil bodies and associated proteins and a middle aqueous phase containing the chymosin, soluble seed proteins and other soluble seed components. Following centrifugation, the aqueous phase was removed and clarified by filtration. The clarified extract was adjusted to a pH of 2.0 by addition of sulfuric acid and allowed to sit for several minutes and then readjusted to pH 5.6 with aqueous ammonia. The extract was then centrifuged at 10,000 x g to remove precipitated proteins and the soluble supernatant phase recovered. The low pH-treated extract was diluted with water to a conductivity of approximately 9.5 mmohs and then loaded on to an anion exchange column containing approximately 30 mls of DEAE-cellulose previously equilibrated with 0.5% sodium benzoate, 0.379% NaCl, pH 5.6. After loading, the column was washed with approximately 200 mls of 0.5% sodium benzoate, 0.379% NaCl, pH 5.6 and then eluted with 110 mls of 0.5% sodium benzoate, 10% NaCl, pH 5.6. The eluate from the anion exchange step was loaded on to a gel filtration column containing G25 sephadex (Amersham-Pharmacia) equilibrated with 25 mM sodium phosphate, 1 M ammonium sulfate, pH 5.6. Fifty mls of the eluate from this column was passed through a 0.22 um filter and then loaded on to a hydrophobic interaction column containing 4.6 mls of butyl sepharose (Fast Flow, Amersham-Pharmacia) previously equilibrated with 25 mM sodium phosphate, 1 M ammonium sulfate, pH 5.6. After loading, the column was washed with 20 mls of 25 mM sodium phosphate, 1 M ammonium sulfate, pH 5.6 followed by 75 mls of 25 mM sodium phosphate, 0.55 M ammonium sulfate, pH 5.6. Purified chymosin was eluted from the column with 24 mls of 25 mM sodium phosphate, 0.1 M ammonium sulfate, pH 5.6. Figure 5 shows a SDS-polyacrylamide gel showing progressive purification of chymosin obtained from transgenic seeds of Brassica napus as described above. Lane 1, aqueous phase from total seed extract; lane 2 pH-treated extract; lane 3, DEAE-cellulose eluate; lane 4, purified chymosin eluted from butyl sepharose.

While the present invention has been described with reference to what are presently considered to be the preferred examples, it is to be understood that the invention is not limited to the disclosed examples.

**TABLE 1**

| | | | |
|---|---|---|---|
| mg of seed extract | 0.5 | 1.0 | 2.0 |
| ng of pro-chymosin in seed extract | 21 | 47 | 88 |
| level of expression (% of protein) | 4.2 | 4.7 | 4.4 |
| Average level of expression (% of protein ) | | 4.43 | |

## Claims

1. A method for the production of chymosin in a plant seed comprising:
a) introducing into a plant cell a chimeric nucleic acid sequence molecule comprising in the 5' to 3' direction of transcription:
1) a first nucleic acid sequence capable of regulating transcription in said plant cell operatively linked to;
2) a second nucleic acid sequence encoding a chymosin polypeptide operatively linked to;
3) a third nucleic acid sequence capable of terminating transcription in said plant cell;
b) growing said plant cell into a mature plant capable of setting seed;
c) obtaining seed from the mature plant wherein said seed contains chymosin; and
d) isolating said chymosin from said seed
wherein isolating said chymosin from said seed comprises:
(i) crushing of the plant seed to obtain crushed plant seed;
(ii) fractionating the crushed plant seed into an oil fraction, aqueous fraction and a fraction comprising insoluble material;
(iii) contacting the aqueous fraction with a protein binding resin; and
(iv) recovering the chymosin from the protein binding resin.

2. The method according to claim 1 wherein said first nucleic sequence capable of regulating transcription in said plant cell is a seed-specific promoter.

3. The method according to claim 2 wherein said seed-specific promoter is a phaseolin promoter.

4. A method according to any one of claims 1 to 3 wherein at least 0.5% (w/w) of the total seed protein is chymosin.

5. The method according to any one of claims 1 to 4 wherein the second nucleic acid sequence encoding a chymosin polypeptide comprises a nucleic acid sequence encoding a chymosin pro-peptide, a nucleic acid sequence encoding a chymosin pre-peptide or a nucleic acid sequence encoding chymosin pre-pro-peptide.

6. The method according to any one of claims 1 to 5 wherein the second nucleic acid sequence encoding a chymosin polypeptide further comprises a nucleic acid sequence encoding a plant signal sequence.

7. The method according to claim 6 wherein the plant signal sequence is a tobacco PR-S sequence.

8. The method according to claim 7 wherein the nucleic acid sequence encoding chymosin linked to a PR-S signal sequence comprises a nucleic acid sequence as in SEQ.ID.NO.:1.

9. The method according to any one of claims 1 to 8 wherein said third nucleic acid sequence is a phaseolin terminator.

10. The method according to any one of claims 1 to 8 wherein the chymosin is a mammalian chymosin obtainable from a bovine, sheep or goat source.

11. The method according to claim 5 wherein codon usage for said nucleic acid sequence encoding chymosin, chymosin pro-peptide, chymosin pre-peptide and chymosin pre-pro-peptide has been optimized for use in plants.

12. The method according to any one of claims 1 to 11 wherein said plant is selected from the group of plants consisting of soybean (*Glycine max),* rapeseed (*Brassica napus, Brassica campestris*)*,* sunflower (*Helianthus annuus*)*,* cotton (*Gossypium hirsutum*)*,* corn *(Zea mays),* tobacco (*Nicotiana tabacum*)*,* alfalafa *(Medicago sativa),* wheat *(Triticum sp.),* barley (*Hordeum vulgare*)*,* oats (*Avena sativa* L.), sorghum (*Sorghum bicolor*)*,* Arabidopsis thaliana, potato (*Solanum sp*.)*,* flax/linseed *(Linum usitatissimum*)*,* safflower (*Carthamus tinctorius*)*,* oil palm *(Elaeis guineensis),* groundnut (*Arachis hypogaea*)*,* Brazil nut *(Bertholletia excelsa)* coconut *(Cocos nucifera),* castor *(Ricinus communis),* coriander (*Coriandrum sativum*)*,* squash (*Cucurbita maxima*)*,* jojoba *(Simmondsia chinensis)* and rice *(Oryza sativa).*

13. The method according to any one of claims 1 to 12 wherein at least 1% (w/w) of said total seed protein is chymosin.

14. The method according to any one of claims 1 to 12 wherein at least 2% (w/w) of said total seed protein is chymosin.

15. The method according to any one of claims 1 to 12 wherein at least 4% (w/w) of said total seed protein is chymosin.

16. A method for the production of plant seeds containing at least 0.5% (w/w) chymosin in the total seed protein comprising:
(a) introducing into each of at least two plant cells a chimeric nucleic acid sequence molecule comprising in the 5' to 3' direction of transcription:
1) a first nucleic acid sequence which is a seed-specific promoter and is capable of regulating transcription in said plant cell operatively linked to;
2) a second nucleic acid sequence encoding a chymosin polypeptide operatively linked to;
3) a third nucleic acid sequence capable of terminating transcription in said plant cell;
(b) growing each plant cell into a mature plant capable of setting seed;
(c) obtaining seed from each mature plant;
(d) detecting the levels of chymosin in the seed of each plant obtained in step (c) or in the seed of a plant generated from the seed of a plant obtained in step (c); and
(e) selecting plants that contain at least 0.5% (w/w) chymosin in the total seed protein.

17. A method according to claim 1 wherein said protein binding resin is a hydrophobic interaction resin.

18. A method according to claim 17 further comprising using an ion exchange resin to further purify the chymosin.

19. Plant seed comprising heterologously expressed chymosin wherein said heterologously expressed chymosin is at least 0.5% (w/w) of the total seed protein.

20. A plant capable of setting seed comprising heterologously expressed chymosin wherein said heterologously expressed chymosin is at least 0.5% (w/w) of the total seed protein.

## Patentansprüche

1. Verfahren zur Herstellung von Chymosin in einem Pflanzensamen, bei dem man:
a) in eine Pflanzenzelle ein chimäres Nukleinsäuresequenzmolekül einführt, welches in der Transkriptionsrichtung 5' zu 3':
1) eine erste Nukleinsäuresequenz umfaßt, die in der Lage ist, die Transkription in dieser Pflanzenzelle zu regulieren, und welche funktionsfähig verbunden ist mit
2) einer zweiten Nukleinsäuresequenz, die ein Chymosin-Polypeptid codiert und die funktionsfähig verbunden ist mit
3) einer dritten Nukleinsäuresequenz, die in der Lage ist, die Transkription in dieser Pflanzenzelle zu stoppen,
b) die Pflanzenzelle zu einer reifen Pflanze anzieht, die in der Lage ist, Samen zu bilden,
c) Samen von der reifen Pflanze gewinnt, wobei der Samen Chymosin enthält, und
d) das Chymosin von dem Samen isoliert,
wobei man bei dem Isolieren des Chymosin von dem Samen:
i) den Pflanzensamen zerkleinert, um zerkleinerten Pflanzensamen zu erhalten,
(ii) den zerkleinerten Pflanzensamen in eine Ölfraktion, eine wäßrige Fraktion und eine Fraktion, die unlösliches Material umfaßt, fraktioniert,
(iii) die wäßrige Fraktion mit einem Protein bindenden Harz in Kontakt bringt und
(iv) das Chymosin von dem Protein bindenden Harz zurückgewinnt.

2. Verfahren nach Anspruch 1, wobei die erste Nukleinsäuresequenz, die in der Lage ist, die Transkription in der Pflanzenzelle zu regulieren, ein samenspezifischer Promotor ist.

3. Verfahren nach Anspruch 2, wobei der samenspezifische Promotor ein Phaseolin-Promotor ist.

4. Verfahren nach einem der Ansprüche 1 bis 3, wobei wenigstens 0,5% (m/m) des gesamten Samenproteins Chymosin ist.

5. Verfahren nach einem der Ansprüche 1 bis 4, wobei die zweite Nukleinsäuresequenz, die ein Chymosin-Polypeptid codiert, eine Nukleinsäuresequenz, die ein Chymosin-Propeptid codiert, eine Nukleinsäuresequenz, die ein Chymosin-Präpeptid codiert, oder eine Nukleinsäuresequenz, die ein Chymosin-Präpropeptid codiert, umfaßt.

6. Verfahren nach einem der Ansprüche 1 bis 5, wobei die zweite Nukleinsäuresequenz, die ein Chymosin-Polypeptid codiert, weiterhin eine Nukleinsäuresequenz umfaßt, die eine pflanzliche Signalsequenz codiert.

7. Verfahren nach Anspruch 6, wobei die pflanzliche Signalsequenz eine Tabak-PR-S-Sequenz ist.

8. Verfahren nach Anspruch 7, wobei die Nukleinsäuresequenz, die Chymosin verbunden mit einer PR-S-Signalsequenz codiert, eine Nukleinsäuresequenz umfaßt, wie die in SEQ.ID.NO.:1.

9. Verfahren nach einem der Ansprüche 1 bis 8, wobei die dritte Nukleinsäuresequenz ein Phaseolin-Terminator ist.

10. Verfahren nach einem der Ansprüche 1 bis 8, wobei das Chymosin ein Säugetier-Chymosin ist, das aus einer Rinder-, Schafs- oder Ziegenquelle erhältlich ist.

11. Verfahren nach Anspruch 5, wobei die Verwendung von Codons für die Nukleinsäuresequenz, die Chymosin, Chymosin-Propeptid, Chymosin-Präpeptid und Chymosin-Präpropeptid codiert, für die Verwendung in Pflanzen optimiert wurde.

12. Verfahren nach einem der Ansprüche 1 bis 11, wobei die Pflanze ausgewählt ist aus der Gruppe von Pflanzen, die besteht aus Sojabohne *(Glycine max),* Rapssamen (*Brassica napus, Brassica campestris*), Sonnenblume (*Helianthus annuus*), Baumwolle (*Gossypium hirsutum*), Mais *(Zea mays),* Tabak (Nicotiana tabacum), Alfalafa *(Medicago sativa),* Weizen *(Triticum sp.),* Gerste (*Hordeum vulgare*), Hafer (*Avena sativa L*.), Hirse (*Sorghum bicolor*), Arabidopsis thaliana, Kartoffel (*Solanum sp*.), Flachs/Leinsamen *(Linum usitatissimum),* Carthamus (*Carthamus tinctorius*), Ölpalme *(Elaeis guineensis),* Erdnuß (*Arachis hypogaea*), Brasilnuß *(Bertholletia excelsa),* Kokosnuß *(Cocos nucifera),* Rizinus (*Ricinus communis),* Koriander (*Coriandrum sativum*), Kürbis (*Cucurbita maxima*), Jojoba *(Simmondsia chinensis)* und Reis (*Oryza sativa).*

13. Verfahren nach einem der Ansprüche 1 bis 12, wobei wenigstens 1% (m/m) des gesamten Samenproteins Chymosin ist.

14. Verfahren nach einem der Ansprüche 1 bis 12, wobei wenigstens 2% (m/m) des gesamten Samenproteins Chymosin ist.

15. Verfahren nach einem der Ansprüche 1 bis 12, wobei wenigstens 4% (m/m) des gesamten Samenproteins Chymosin ist.

16. Verfahren zur Herstellung von Pflanzensamen, die wenigstens 0,5% (m/m) Chymosin im gesamten Samenprotein enthalten, bei dem man:
(a) in jede von wenigstens zwei Pflanzenzellen ein chimäres Nukleinsäuresequenzmolekül einführt, welches in der Transkriptionsrichtung 5' zu 3':
1) eine erste Nukleinsäuresequenz umfaßt, die ein samenspezifischer Promotor ist und in der Lage ist, die Transkription in der Pflanzenzelle zu regulieren, und welche funktionsfähig verbunden ist mit
2) einer zweiten Nukleinsäuresequenz, die ein Chymosin-Polypeptid codiert und die funktionsfähig verbunden ist mit
3) einer dritten Nukleinsäuresequenz, die in der Lage ist, die Transkription in dieser Pflanzenzelle zu stoppen,
(b) jede Pflanzenzelle zu einer reifen Pflanze anzieht, die in der Lage ist, Samen zu bilden,
(c) Samen von jeder reifen Pflanze gewinnt,
(d) die Gehalte an Chymosin in dem Samen von jeder Pflanze, die in Stufe (c) erhalten wurde, oder in dem Samen einer Pflanze, die aus dem Samen einer Pflanze, die in Stufe (c) erhalten wurde, erzeugt wurde, erfaßt und
(e) Pflanzen auswählt, die wenigstens 0,5% (m/m) Chymosin im gesamten Samenprotein enthalten.

17. Verfahren nach Anspruch 1, wobei das Protein bindende Harz ein Harz mit hydrophober Wechselwirkung ist.

18. Verfahren nach Anspruch 17, bei dem man des weiteren ein Ionenaustauscherharz verwendet, um das Chymosin weiter aufzureinigen.

19. Pflanzensamen, umfassend heterolog exprimiertes Chymosin, wobei das heterolog exprimierte Chymosin wenigstens 0,5% (m/m) des gesamten Samenproteins ausmacht.

20. Pflanze, die in der Lage ist, Samen zu bilden, die heterolog exprimiertes Chymosin enthalten, wobei das heterolog exprimierte Chymosin wenigstens 0,5% (m/m) des gesamten Samenproteins ausmacht.

## Revendications

1. Procédé pour la production de chymosine dans une graine d'une plante, comprenant les étapes consistant à :
a) introduire dans une cellule végétale une molécule de séquence d'acide nucléique chimère comprenant, dans le sens 5' à 3' de la transcription:
1) une première séquence d'acide nucléique capable de réguler la transcription dans ladite cellule végétale, liée de manière fonctionnelle ;
2) une deuxième séquence d'acide nucléique codant pour un polypeptide de chymosine, liée de manière fonctionnelle ;
3) une troisième séquence d'acide nucléique capable de terminer la transcription dans ladite cellule végétale ;
b) faire croître ladite cellule végétale en une plante mature capable d'émettre des graines ;
c) obtenir des graines à partir de la plante mature, lesdites graines contenant de la chymosine ; et
d) isoler ladite chymosine desdites graines,
dans lequel l'isolement de ladite chymosine desdites graines comprend les étapes consistant à :
(i) broyer les graines de la plante pour obtenir des graines broyées de la plante ;
(ii) fractionner les graines broyées de la plante en une fraction d'huile, une fraction aqueuse et une fraction comprenant une matière insoluble ;
(iii) mettre en contact la fraction aqueuse avec une résine fixant les protéines ; et
(iv) recueillir la chymosine à partir de la résine fixant les protéines.

2. Procédé suivant la revendication 1, dans lequel ladite première séquence d'acide nucléique capable de réguler la transcription dans ladite cellule végétale est un promoteur spécifique des graines.

3. Procédé suivant la revendication 2, dans lequel ledit promoteur spécifique des graines est un promoteur de phaséoline.

4. Procédé suivant l'une quelconque des revendications 1 à 3, dans lequel une quantité d'au moins 0,5 % (en poids/poids) des protéines totales des graines est constituée de chymosine.

5. Procédé suivant l'une quelconque des revendications 1 à 4, dans lequel la seconde séquence d'acide nucléique codant pour un polypeptide de chymosine comprend une séquence d'acide nucléique codant pour un propeptide de chymosine, une séquence d'acide nucléique codant pour un prépeptide de chymosine ou une séquence d'acide nucléique codant pour pré-propeptide de chymosine.

6. Procédé suivant l'une quelconque des revendications 1 à 5, dans lequel la deuxième séquence d'acide nucléique codant pour un polypeptide de chymosine comprend en outre une séquence d'acide nucléique codant pour une séquence signal de plante.

7. Procédé suivant la revendication 6, dans lequel la séquence signal de plante est une séquence PR-S du tabac.

8. Procédé suivant la revendication 7, dans lequel la séquence d'acide nucléique codant pour la chymosine liée à une séquence signal PR-S comprend une séquence d'acide nucléique indiquée dans la SEQ ID N° 1.

9. Procédé suivant l'une quelconque des revendications 1 à 8, dans lequel ladite troisième séquence d'acide nucléique est une séquence de terminateur de phaséoline.

10. Procédé suivant l'une quelconque des revendications 1 à 8, dans lequel la chymosine est une chymosine de mammifère pouvant être obtenue à partir d'une source bovine, ovine ou caprine.

11. Procédé suivant la revendication 5, dans lequel l'utilisation des codons pour ladite séquence d'acide nucléique codant pour la chymosine, le propeptide de chymosine, le prépeptide de chymosine ou le pré-propeptide de chymosine a été optimisée pour l'utilisation dans des plantes.

12. Procédé suivant l'une quelconque des revendications 1 à 11, dans lequel ladite plante est choisie dans le groupe de plantes consistant en le soja *(Glycine max),* le colza (*Brassica napus, Brassica campestris*)*,* le tournesol (*Helianthus annuus*), le cotonnier (*Gossypium hirsutum*), le maïs *(Zea mays*), le tabac (*Nicotiana tabacum*), la luzerne *(Medicago sativa*), le blé *(Triticum sp.),* l'orge (*Hordeum vulgare*), l'avoine (*Avena sativa L*.), le sorgho (*Sorghum bicolor*), *Arabidopsis thaliana,* la pomme de terre (*Solanum sp*.), le lin *(Linum usitatissimum),* le carthame (*Carthamus tinctorius*), le palmier à huile *(Elaeis guineensis),* l'arachide (*Arachis hypogaea*), la noix du Brésil *(Bertholletia excelsa),* le cocotier *(Cocos nucifera),* le ricin *(Ricinus communis),* la coriandre (*Coriandrum sativum*), la courge (*Cucurbita maxima*), le jojoba *(Simmondsia chinensis)* et le riz *(Oryza sativa).*

13. Procédé suivant l'une quelconque des revendications 1 à 12, dans lequel une quantité d'au moins 1 % (en poids/poids) desdites protéines totales des graines est constituée de chymosine.

14. Procédé suivant l'une quelconque des revendications 1 à 12, dans lequel une quantité d'au moins 2 % (en poids/poids) desdites protéines totales des graines est constituée de chymosine.

15. Procédé suivant l'une quelconque des revendications 1 à 12, dans lequel une quantité d'au moins 4 % (en poids/poids) desdites protéines totales des graines est constituée de chymosine.

16. Procédé pour la production de graines d'une plante contenant au moins 0,5 % (en poids/poids) de chymosine dans les protéines totales des graines, comprenant les étapes consistant à :
(a) introduire dana chacune d'au moins deux cellules végétales une molécule de séquence d'acide nucléique chimère comprenant, dans le sens 5' à 3' de la transcription :
1) une première séquence d'acide nucléique qui est un promoteur spécifique des graines et qui est capable de réguler la transcription dans ladite cellule végétale, liée de manière fonctionnelle ;
2) une deuxième séquence d'acide nucléique codant pour un polypeptide de chymosine, liée de manière fonctionnelle ;
3) une troisième séquence d'acide nucléique capable de terminer la transcription dans ladite cellule végétale ;
(b) faire croître chaque cellule végétale en une plante mature capable d'émettre des graines ;
(c) obtenir des graines à partir de chaque plante mature ;
(d) détecter les taux de chymosine dans les graines de chaque plante obtenues dans l'étape (c) ou dans les graines d'une plante engendrée à partir des graines d'une plante obtenue dans l'étape (c) ; et
(e) sélectionner les plantes qui contiennent au moins 0,5 % (en poids/poids) de chymosine dans les protéines totales des graines.

17. Procédé suivant la revendication 1, dans lequel ladite résine fixant les protéines est une résine d'interaction hydrophobe.

18. Procédé suivant la revendication 17, comprenant en outre l'utilisation d'une résine échangeuse d'ions pour purifier davantage la chymosine.

19. Graine de plante comprenant de la chymosine exprimée de manière hétérologue, dans laquelle ladite chymosine exprimée de manière hétérologue représente au moins 0,5 % (en poids/poids) des protéines totales des graines.

20. Plante capable d'émettre des graines comprenant de la chymosine exprimée de manière hétérologue, dans laquelle ladite chymosine exprimée de manière hétérologue représente au moins 0,5 % (en poids/poids) des protéines totales des graines.
